# EUROPEAN PATENT APPLICATION

(11) **EP 4 685 237 A1**
(43) Date of publication of application: **28.01.2026**
(21) Application number: 24382824.1
(22) Date of filing: 26.07.2024
(51) Int. Cl.: C12P 17/12, C07D 209/94, A61P 1/00, A61K 31/403

(54) **PROCESS AND INTERMEDIATES FOR THE PREPARATION OF ETRASIMOD AND RELATED COMPOUNDS**

(71) Applicant: CURIA SPAIN, S.A.U., 47151 Boecillo, Valladolid (ES)
(72) Inventor: LORENTE BONDE-LARSEN, Antonio, E-47151 Boecillo - Valladolid (ES); MESSINA, Ivano, E-47151 Boecillo - Valladolid (ES); PAZOS AGUETE, Gonzalo, E-47151 Boecillo - Valladolid (ES)
(74) Representative: ABG Intellectual Property Law, S.L.

(57) **Abstract**

The present invention relates to an efficient and industrially applicable process and intermediates for the preparation of etrasimod and related compounds.

## Description

### Field of the Invention

The invention belongs to the field of drug synthesis, and more particularly relates to an efficient and industrially applicable process for the preparation of etrasimod and related compounds and to intermediates useful therefor.

### Background of the Invention

Etrasimod and other substituted 2-(1,2,3,4-tetrahydrocyclopenta[b]indol-3-yl)acetic acid derivatives have been disclosed in the prior art as useful in the treatment of sphingosine-1-phosphate (S1P) receptor-associated disorders, for example, psoriasis, rheumatoid arthritis, Crohn's disease, transplant rejection, multiple sclerosis, systemic lupus erythematosus, ulcerative colitis, type I diabetes, acne, microbial infections or diseases and viral infections or diseases.

Specifically, etrasimod (also known as APD334) is the international non-proprietary name (INN) or common name of (*R*)-2-(7-(4-cyclopentyl-3-(trifluoromethyl)benzyloxy)-1,2,3,4-tetrahydrocyclopenta[b]indol-3-yl)acetic acid, a potent, selective and orally available antagonist of the sphingosine-1-phosphate-1 (S1P1) receptor, which was approved on October 2023 by the FDA under the brand name VELSIPITY for the treatment of adults with moderately to severely active ulcerative colitis. The active ingredient in VELSIPITY is the L-arginine salt of etrasimod (commonly simply referred to as etrasimod arginine) and it has the following chemical structure:

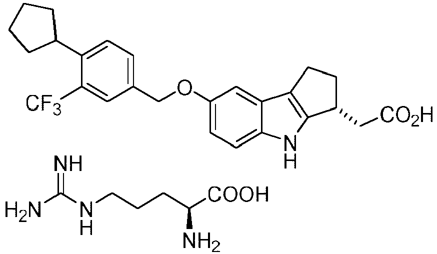

### Etrasimod arginine

Several synthetic processes for the preparation of this type of compounds and intermediates thereof have been disclosed.

WO 2010011316 A1 (example 1.4: compound 12) discloses a process for preparing racemic etrasimod followed by resolution via chiral HPLC to obtain both enantiomers. However, chiral separation by HPLC is not suitable at an industrial scale, specially owing to the high cost of the stationary phase (packing materials).

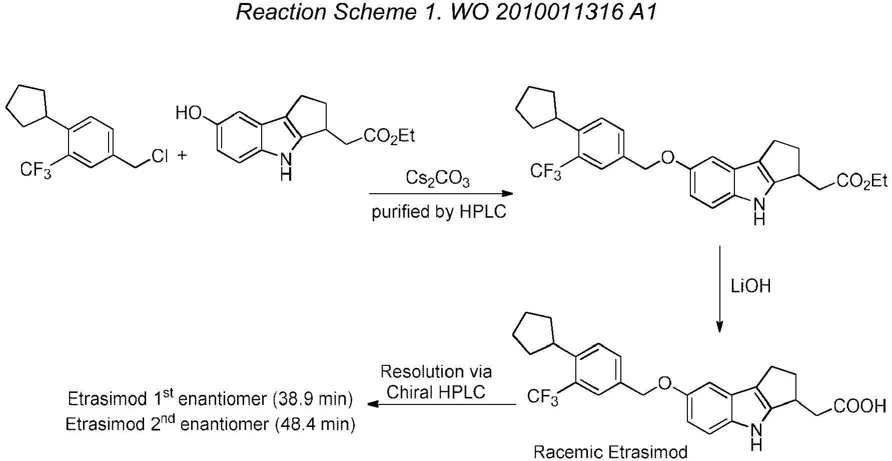

WO 2011094008 A1 (example 7: compound of formula (Ia)) describes the preparation of etrasimod through a process whose last step is the enantioselective enzymatic hydrolysis of the ester rac-ethyl 2-(7-(4-cyclopentyl-3-(trifluoromethyl)benzyloxy)-1,2,3,4-tetrahydrocyclopenta[b]indol-3-yl)acetate which provides a mixture containing etrasimod with the desired (R) configuration and the (S)-ester as an impurity. The process then requires adding seeds of the L-arginine salt of (R)-etrasimod to the mixture so as to induce the crystallization of etrasimod arginine.

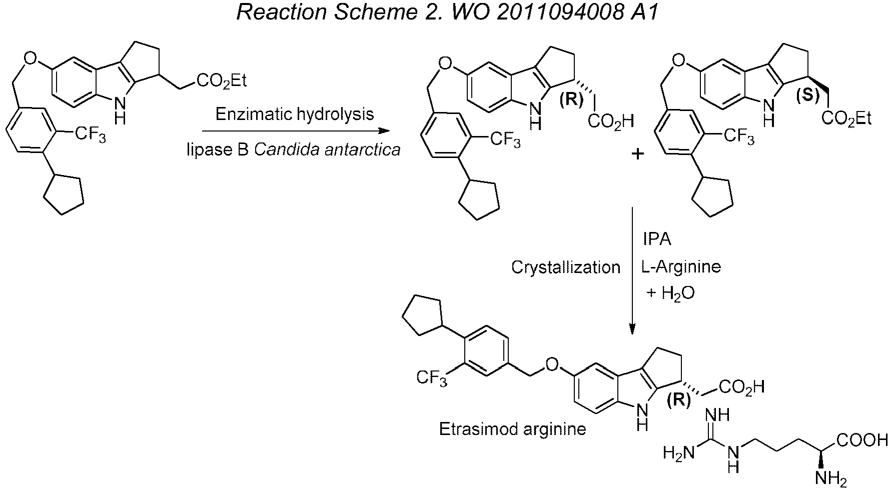

Crystallization has been widely used for product separation and purification in the fields of food, pharmaceutical and chemical industry. Seeding is a significant but challenging task in optimizing crystallization process operation, product quality, and process efficiency. Traditionally, seed crystals are added from an external source, generally from a milled crystals or from previous batch products. This seeding mode is powerful and multifunctional, but the abrupt introduction of seed may destroy the stable environment in crystallizer, hence increasing the probability of inducing the explosive nucleation and introducing impurities and bacteria into crystallizer. Moreover, seed preparation is sometimes complex, costly and time-consuming if high-quality seed is required, and the exact dosage is hard to calculate. Thus, seeding is a powerful but complex step for industrial crystallization process, which includes many parameters that should be taken into account commonly when designing and implementing a seeding strategy. (Zhang, F, Shan, B, Wang, Y et al. (2021) Progress and Opportunities for Utilizing Seeding Techniques in Crystallization Processes. Organic Process Research and Development, 25 (7). pp. 1496-1511. ISSN 1083-6160).

More recently, WO 2016209809 A1 has used this same strategy of enzymatic hydrolysis to prepare a crystalline free-plate habit of etrasimod arginine. The preparation of this allegedly new morphology, however, does not avoid the need of adding the L-arginine salt of (*R*)-etrasimod for seeding (see example 4, methods 1-3) or reworking previously formed crystals by recrystallization (see example 3 in which the four lots of the L-arginine salt of (*R*)-etrasimod described in example 2 were recrystallized).

Besides the intrinsic complexity of the seeding technique in the very last step of an industrial crystallization procedure, the process reported in WO 2011094008 A1 and WO 2016209809 A1 for obtaining etrasimod in the desired (R)-enantiomeric form (and its L-arginine salt) has important drawbacks in terms of its synthetic efficacy and implementation at an industrial scale:
- The fact that the desired chirality is introduced in the structure in the last step of the synthesis results in an elevated overall process cost (including environmental, operating and material costs) since all the previous reactions take place with racemic intermediates (i.e. 50:50 mixtures of desired and undesired enantiomer), thus requiring a higher amount of materials and generating more waste in comparison to a process involving transformations of earlier intermediates already having the desired chirality (see example 19 herein).
- During the salt formation the final product precipitates from a reaction mixture containing over 50% of the (S)-isomer ester as impurity, which may interfere both with the formation of a crystalline and pure product and with the crystallization yield (in the very last step of the synthetic step of the route).
- The non-hydrolyzed (S)-isomer ester can hydrolyze into the non-desired (S)-enantiomer of etrasimod during the final isolation, which would decrease the enantiomeric excess.

It is therefore necessary to develop new processes for obtaining etrasimod and related compounds, as well as key intermediates in their synthesis, which overcome all or part of the problems associated with the known processes belonging to the state of the art.

### Summary of the Invention

The present invention solves the aforementioned needs by the provision of a simple, cost-effective and industrially applicable process for the preparation of enantiomeric substituted 2-(1,2,3,4-tetrahydrocyclopenta[b]indol-3-yl)acetic acid derivatives such as etrasimod and to intermediates useful therefor.

Specifically, after conducting extensive experimentation, the inventors have surprisingly found that chiral substituted 2-(1,2,3,4-tetrahydrocyclopenta[b]indol-3-yl)acetic acid derivatives such as etrasimod can be obtained through a process involving the enantioselective enzymatic hydrolysis of early synthetic intermediates through the following synthetic schemes:

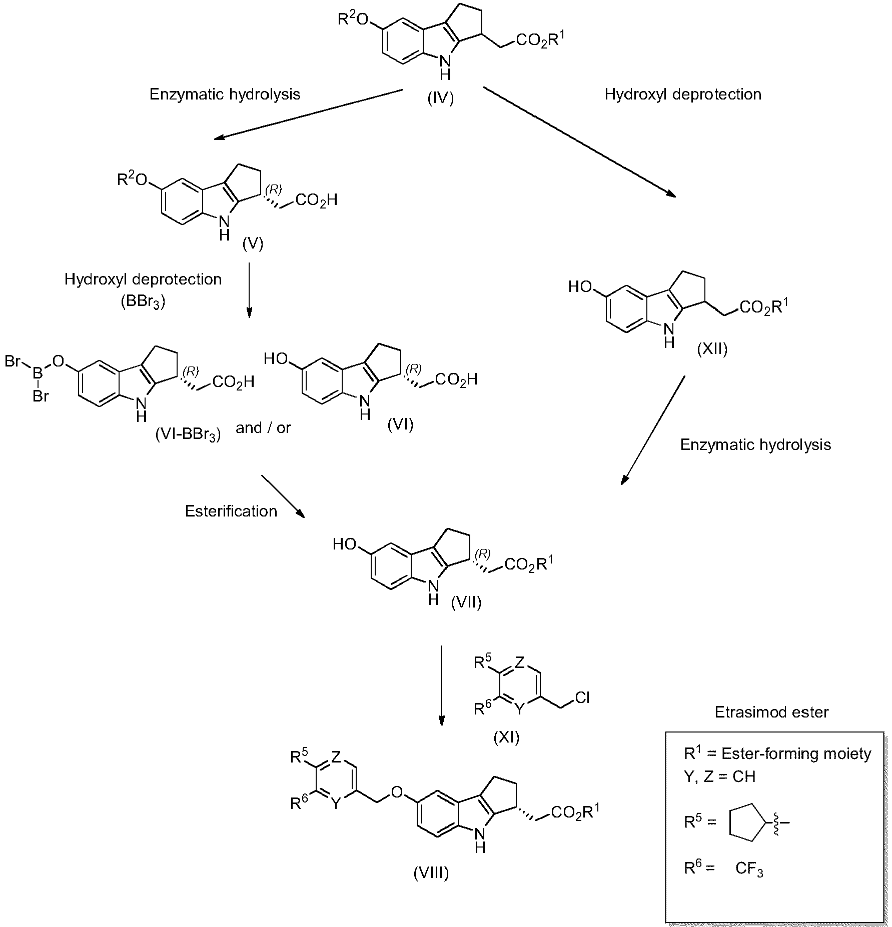

It is worth mentioning that racemic esters falling within general formula (IV) or (XII) such as ethyl 2-(7-hydroxy-1,2,3,4-tetrahydrocyclopenta[b]indol-3-yl)acetate have been used in the prior art methods disclosed for the synthesis of etrasimod in WO 2010011316 A1 (example 1.1, step D) and, even in WO 2011094008 A1 (example 5) which indeed also describes an enantioselective enzymatic hydrolysis but mandatorily as last step. However, to the best knowledge of the inventors, the possibility of introducing chirality through enantioselective enzymatic hydrolysis in such early synthetic intermediates has not been envisaged in the state of the art.

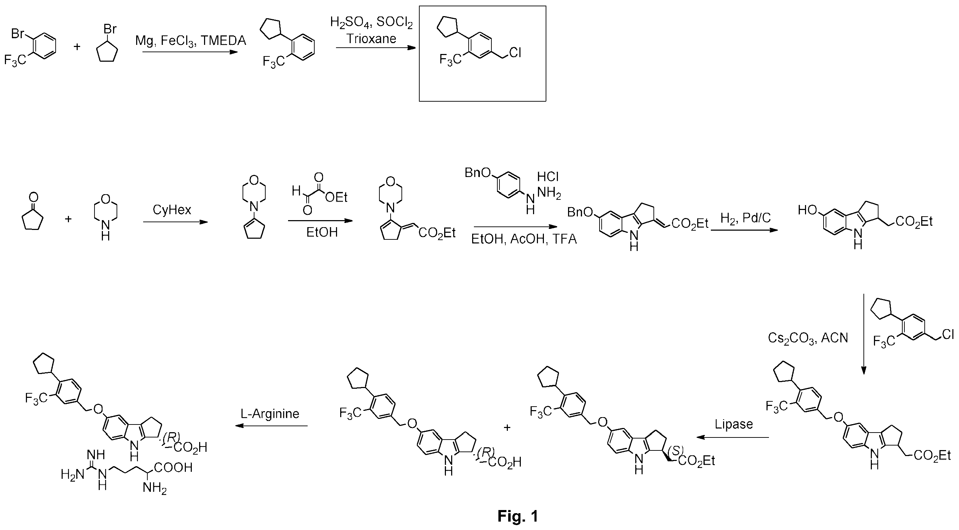

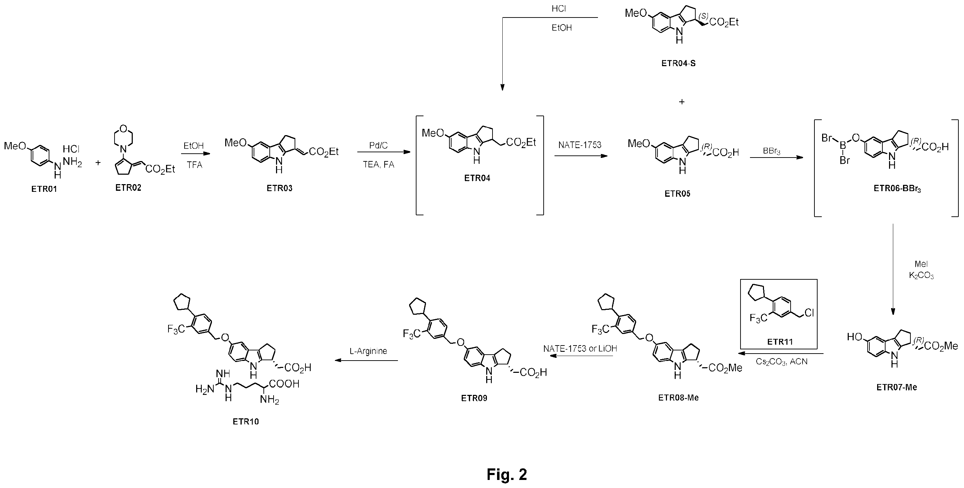

As explained in the background section, the processes of enantioselective hydrolysis of racemic compounds should be implemented as early as possible in a synthetic route since it is of outmost importance, especially for large-scale production, to use the minimum possible amount of materials (reagents, reactants, solvents ...), energy and other resources. Reference is made to example 19 which shows a comparison between the route for preparing etrasimod arginine disclosed in WO 2011094008 A1 (depicted in Figure 1) and the one proposed in the present invention according to one embodiment thereof (depicted in Figure 2). As may be appreciated, when compared to the prior art process, the innovative synthetic approach now described herein results in better overall molar yield (14% vs 10%) and better overall weight yield (50% vs 25%). Moreover, in the process now proposed, if desired, the (S)-isomer ester that remains non-hydrolyzed in the enantioselective hydrolysis may be epimerized and subjected to a second enantioselective hydrolysis so as to reach yields above 50% in this step (example 7 for instance reports a yield of 59%).

Thus, in a first aspect, the invention is directed to a process for preparing a compound of Formula (VIII) or a salt or solvate thereof 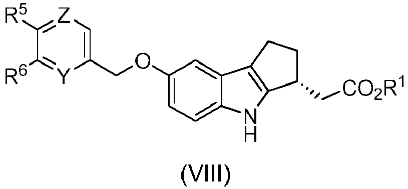 wherein
R¹ is an organic group forming an ester with the carboxyl group to which it is attached;
Y is N or CR³;
Z is N or CR⁴; and
R³, R⁴, R⁵ and R⁶ are each independently selected from the group consisting of H, C₁-C₆ alkyl, C₂-C₆ alkenyl, C₃-C₇ cycloalkyl, C₆-C₁₀ aryl, (C₆-C₁₀)aryl(C₁-C₆)alkyl, 3- to 10-membered heterocyclyl, 3- to 10-membered heteroaryl, C₁-C₆ alkoxy, C₁-C₆ alkylamino, C₁-C₆ alkylsulfonyl, C₁-C₆ alkylthio, carboxamide, cyano, C₃-C₇ cycloalkoxy, C₁-C₆ haloalkoxy, C₁-C₆ haloalkyl, and halogen, wherein said C₁-C₆ alkyl and C₁-C₆ alkoxy are each optionally substituted with one or two substituents selected from C₃-C₇ cycloalkyl and halogen;
from a compound of Formula (IV) or a salt or solvate thereof 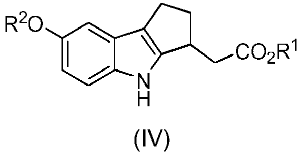
wherein
R¹ is as defined for the compound of Formula (VIII); and
R² is selected from the group consisting of C₁-C₆ alkyl and C₃-C₇ cycloalkyl, wherein said C₁-C₆ alkyl, and C₃-C₇ cycloalkyl are each optionally substituted with one or more substituents selected from the group consisting of C₁₋₆ alkyl, C₁₋₆ alkoxy, halogen, C₁₋₆ haloalkyl, C₁₋₆ haloalkoxy, nitro and cyano;
said process comprising:
   (a1) enantioselective enzymatic hydrolysis of the compound of Formula (IV) or a salt or solvate thereof in the presence of a lipase selected *Thermomyces lanuginosus* lipase and *Candida antarctica* lipase B to provide a compound of Formula (V) or a salt or solvate thereof 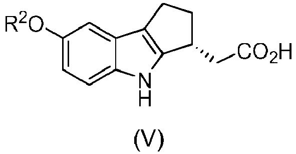 wherein
      R² is as defined for the compound of Formula (IV);
   (b1) hydroxyl deprotection of the compound of Formula (V) or a salt or solvate thereof with boron tribromide, followed by quenching with a tertiary alcohol, to provide a compound of Formula (VI-BBr₃) and/or (VI) or a salt or solvate thereof 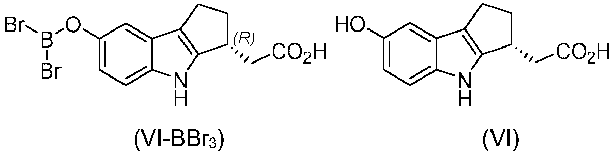 esterification of the compound of Formula (VI-BBr₃) and/or (VI) or a salt or solvate thereof in basic media to provide a compound of Formula (VII) or a salt or solvate thereof 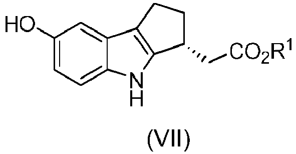 wherein
      R¹ is as defined for the compound of Formula (VIII); and
   (c) reaction of the compound of Formula (VII) or a salt or solvate thereof with a compound of Formula (XI) or a salt or solvate thereof 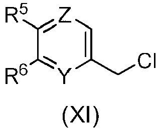 wherein
      Y, Z, R⁵ and R⁶ are as defined for the compound of Formula (VIII);
      to provide the compound of Formula (VIII) or a salt or solvate thereof;
      or said process comprising:
      (a2) hydroxyl deprotection of the compound of Formula (IV) or a salt or solvate thereof in the presence of a hydroxyl deprotection agent to provide a compound of Formula (XII) or a salt or solvate thereof; 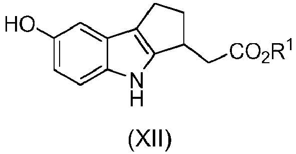 wherein
         R¹ is as defined for the compound of Formula (VIII);
      (b2) enantioselective enzymatic hydrolysis of a compound of Formula (XII) or a salt or solvate thereof in the presence of a lipase selected from *Thermomyces lanuginosus* lipase and *Candida antarctica* lipase B to provide a compound of Formula (VII) or a salt or solvate thereof 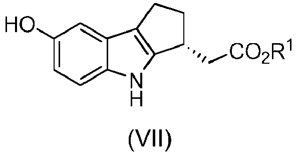 wherein
         R¹ is as defined for the compound of Formula (VIII);
   (c) reaction of the compound of Formula (VII) or a salt or solvate thereof with a compound of Formula (XI) or a salt or solvate thereof 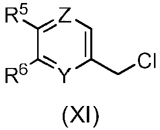 wherein
      Y, Z, R⁵ and R⁶ are as defined for the compound of Formula (VIII);
      to provide the compound of Formula (VIII) or a salt or solvate thereof.

In preferred embodiments, the process of the invention provides a compound of Formula (VIII) which is an ester of etrasimod of formula: 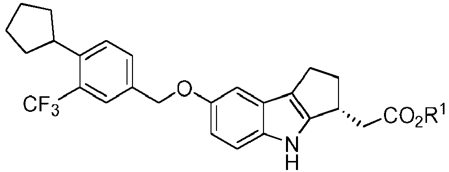 wherein R¹ is a ester-forming moiety i.e. an organic group forming an ester with the carboxyl group to which it is attached.

Compounds of Formula (VIII), and salts or solvates thereof, are advanced intermediates in the synthesis of etrasimod or a salt or solvate thereof and related compounds. Thus, the final making of etrasimod or a salt or solvate thereof and related compounds through the process provided herein for preparing a compound of Formula (VIII) or a salt or solvate thereof is also encompassed within the present invention.

The present invention also provides novel intermediates which may be used for the synthesis of etrasimod through the process provided herein.

In a further aspect, the invention is directed to:

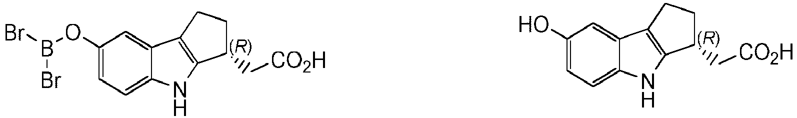

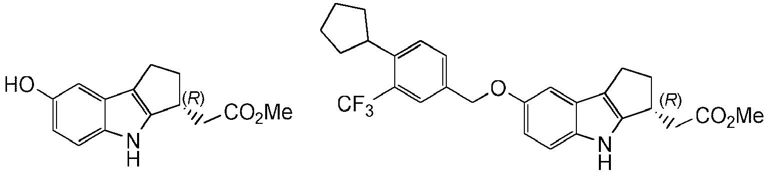

These aspects and preferred embodiments thereof are additionally also defined hereinafter in the detailed description and in the claims.

All the features described in this specification (including the claims, description and drawings) can be combined in any combination, with the exception of combinations of such mutually exclusive features.

### Brief description of Drawings

To better understand the invention, its objects and advantages, the following figures are attached to the specification in which the following is depicted:
**Figure 1** shows a general scheme of the prior art process for preparing etrasimod arginine of WO 2011/094008 A1.
**Figure 2** shows a general scheme of the process for preparing etrasimod arginine according to an embodiment of the present invention comprising enantioselective enzymatic hydrolysis of a compound of Formula (IV) (**ETR04**), hydroxyl deprotection of the compound of Formula (V) (**ETR05**) with BBr₃, basic esterification of the compound of Formula (VI-BBr₃) **(ETR06-BBr₃)** and reaction of the compound of Formula (VII) (**ETR07-Me**) with a compound of Formula (XI) (**ETR11**) to provide the compound of Formula (VIII) (**ETR08**).

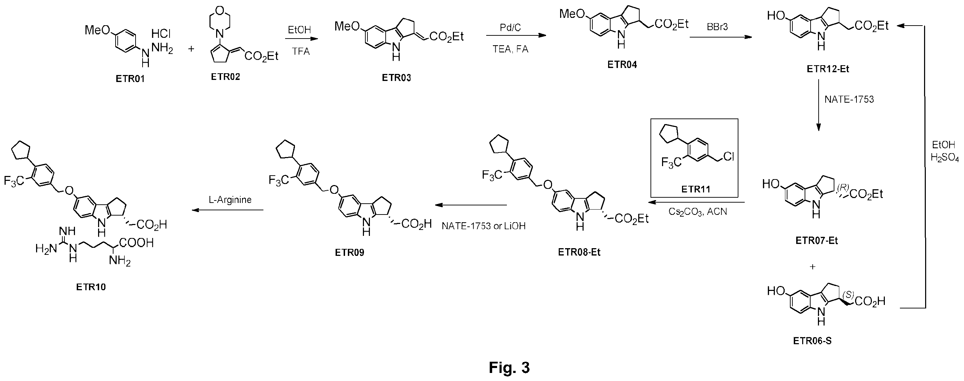

**Figure 3** shows a general scheme of the process for preparing etrasimod arginine according to an embodiment of the present invention comprising hydroxyl deprotection of the compound of Formula (IV) (**ETR04**), enantioselective enzymatic hydrolysis of a compound of Formula (XII) (**ETR12**) and reaction of the compound of Formula (VII) (**ETR07-Et**) with a compound of Formula (XI) (**ETR11**) to provide the compound of Formula (VIII) (**ETR08**).
**Figure 4** shows a SEM imagen of crystalline etrasimod arginine according to example 17 of the present invention.
**Figure 5** shows the PXRD pattern of etrasimod arginine according to example 17 of the present invention.
**Figure 6** shows the DSC analysis of etrasimod arginine according to example 17 of the present invention.
**Figure 7** shows the PXRD pattern of amorphous etrasimod arginine according to example 18 the present invention.

### Detailed Description of the Invention

The present inventors have developed a new process for the preparation of chiral substituted 2-(1,2,3,4-tetrahydrocyclopenta[b]indol-3-yl)acetic acid derivatives such as etrasimod and to intermediates useful therefor. The process of the present invention is simple, reproducible and is well suited for industrial scale.

### Definitions

As used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. The term "comprises" encompasses the terms "consisting essentially of" and "consisting of". Thus, at each occurrence in the present document, the term "comprising" may be replaced with the term "consisting essentially of" or "consisting of". The term "consisting essentially of" means that the specified components represent at least 98wt%, or even at least 99wt%, of the total weight of the corresponding composition, solution or mixture.

As used herein, the term "and/or" includes any and all combinations of one or more of the associated listed items and may be abbreviated as "/".

As used herein, the term "approximately" or "about" as applied to one or more values of interest, refers to a value that is similar to a stated reference value. In certain embodiments, the term "approximately" or "about" refers to a value that can vary up to ± 20 %, preferably within ± 10 %, and more preferably within ± 5 % of the stated reference value. When "approximately" or "about" is used before a numerical range, it applies to the upper and lower range end-points.

Indeed, the skilled person knows that numerical values relating to measurements are subject to measurement errors which place limits on their accuracy. Where terms such as "about" or "approximately" are applied to a particular value (e.g., "about 200 °C" or "approximately 200 °C") or to a range (e.g., "about x to approximately y"), the value or range may be interpreted as being as accurate as the method used to measure it. Unless explicitly stated otherwise, the general convention in the scientific and technical literature may be applied so that the last digit of numerical values preferably indicates the precision of measurement. Thus, unless other error margins are given, the maximum margin is preferably ascertained by applying the rounding-off convention to the last decimal place. For instance, a value of 3.5 preferably has an error margin of 3.45 to 3.54 and a range of 2% to 10% preferably covers a range of 1.5% to 10.4%. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 % to about 5 %" should be interpreted to include not only the explicitly recited values of about 1 % to about 5 %, but also include individual values and sub-ranges within the indicated range. Thus, included in this numerical range are individual values such as 2, 3 and 4 and sub-ranges such as from 1-3, from 2-4, and from 3-5, etc. This same principle applies to ranges reciting only one numerical value. It should also be understood that ranges formed by combination of any of the end points of different disclosed ranges and/or particular values therein are included in the present disclosure.

The term "alkyl" refers to a linear or branched alkane derivative containing from 1 to 6 ("C₁-C₆ alkyl"), preferably from 1 to 3 ("C₁-C₃ alkyl"), carbon atoms and which is bound to the rest of the molecule through a single bond. Illustrative examples of alkyl groups include methyl, ethyl, n-propyl, i-propyl, n-butyl, i-butyl, t-butyl, pentyl, hexyl.

The term "alkenyl" refers to a linear or branched hydrocarbon chain radical containing from 2 to 6 ("C₂-C₆ alkenyl"), preferably from 2 to 3 ("C₂-C₃ alkenyl"), carbon atoms and which contains at least one double bond and is attached to the rest of the molecule by a single bond. Examples of alkenyl groups include ethenyl, propenyl, allyl, butenyl, 1-methyl-2-buten-1-yl, and the like.

The term "cycloalkyl" refers to a radical derived from cycloalkane containing from 3 to 7 ("C₃-C₇ cycloalkyl"), preferably from 3 to 6 ("C₃-C₆ cycloalkyl") carbon atoms. Illustrative examples of cycloalkyl groups include cyclopropyl, cyclobutyl, cyclopentyl, cyclohexyl, etc.

The term "aryl" refers to an aromatic group having between 6 and 10 ("C₆-C₁₀ aryl"), preferably 6 or 10 carbon atoms, comprising 1 or 2 aromatic nuclei, bound by means of a carbon-carbon bond or fused. Illustrative examples of aryl groups include phenyl, naphthyl, diphenyl, indenyl, etc. Preferably, it is phenyl.

The term "arylalkyl" refers to an alkyl group as defined above substituted with an aryl group as defined above, such as e.g. (C₆-C₁₀)aryl(C₁-C₆)alkyl and (C₆-C₁₀)aryl(C₁-C₃)alkyl. Examples of such groups include benzyl, phenylethyl, phenylpropyl, naphthylmethyl, etc. Preferably, it is benzyl.

The term "heterocyclyl" refers to a saturated or partially unsaturated monocyclic or bicyclic system containing from 3 to 10, preferably 5 to 7, ring atoms containing one or more, specifically one, two, three or four ring heteroatoms independently selected from N, O, and S, and the remaining ring atoms being carbon. Illustrative examples of heterocyclyl groups include tetrahydropyran, morpholine, piperazine, piperidine and [1,4]dioxane.

The term "heteroaryl" refers to an aromatic monocyclic or bicyclic system containing from 3 to 10, preferably 5 to 7, ring atoms containing one or more, specifically one, two, three or four ring heteroatoms independently selected from O, N and S, and the remaining ring atoms being carbon. Illustrative examples of heteroaryl groups include pyrrole, furan, thiophene, pyridine and pyrimidine.

The term "alkoxy" designates an alkyl group as defined above having between 1 and 6 carbon atoms ("C₁-C₆ alkoxy"), preferably between 1 and 3 carbon atoms ("C₁-C₃ alkoxy"), linked to the rest of the molecule through oxygen. Examples of alkoxy include methoxy, ethoxy, isopropoxy, tertbutoxy, and the like.

The term "alkylamino" designates an alkyl group as defined above having between 1 and 6 carbon atoms ("C₁-C₆ alkylamino"), preferably between 1 and 3 carbon atoms ("C₁-C₃ alkylamino"), linked to the rest of the molecule through an amino group.

The term "alkylsulfonyl" designates an alkyl group as defined above having between 1 and 6 carbon atoms ("C₁-C₆ alkylsulfonyl"), preferably between 1 and 3 carbon atoms ("C₁-C₃ alkylsulfonyl"), linked to the rest of the molecule through a sulfonyl group.

The term "alkylthio" designates an alkyl group as defined above having between 1 and 6 carbon atoms ("C₁-C₆ alkylthio"), preferably between 1 and 3 carbon atoms ("C₁-C₃ alkylthio"), linked to the rest of the molecule through sulfur.

The term "cycloalkoxy" designates a cycloalkyl group as defined above having between 3 and 7 carbon atoms ("C₃-C₇ cycloalkoxy"), preferably between 3 and 6 carbon atoms ("C₃-C₆ cycloalkoxy"), linked to the rest of the molecule through oxygen.

The term "haloalkoxy" designates alkoxy group as defined above having between 1 and 6 carbon atoms ("C₁-C₆ haloalkoxy"), preferably between 1 and 3 carbon atoms ("C₁-C₃ haloalkoxy"), and having at least one halogen.

The term "haloalkyl" designates an alkyl group as defined above having between 1 and 6 carbon atoms ("C₁-C₆ haloalkyl"), preferably between 1 and 3 carbon atoms ("C₁-C₃ haloalkyl"), and having at least one halogen.

The term "halogen" refers to bromine, chlorine, iodine or fluorine.

As understood in this technical area, there may be a certain degree of substitution in the aforementioned radicals. Therefore, there may be substitution in any of the groups of the present invention. The previous groups can be substituted in one or more available positions with one or more substituents, e.g. one, two or three substituents. Said substituents include, for example and in non-limiting sense, C₁₋₆ alkyl, C₁₋₆ haloalkyl, C₃₋₇ cycloalkyl, C₆-C₁₀ aryl, (C₆-C₁₀)aryl(C₁-C₆)alkyl, 3- to 10-membered heterocyclyl, 3- to 10-membered heteroaryl, halogen, -CN, NO₂, -N(Rₐ)(R_{b}), -OR_{c}, -SR_{d}, -C(O)Rₑ, - C(O)OR_{f}, -C(O)N(R_{g})(Rₕ), -OC(O)Rᵢ; wherein Rₐ, R_{b}, R_{c}, R_{d}, Rₑ, R_{f}, R_{g}, Rₕ and Rᵢ are independently selected from hydrogen, C₁-C₆ alkyl, C₁₋₆ haloalkyl, C₆-C₁₀ aryl, (C₆-C₁₀)aryl(C₁-C₆)alkyl, 3- to 10-membered heterocyclyl, and 3- to 10-membered heteroaryl.

The invention also provides "salts" of the compounds described in the present description. By way of illustration, said salts can be acid addition salts, base addition salts or metal salts, and can be synthesized from the parent compounds containing a basic or acid moiety by means of conventional chemical processes known in the art. Such salts are generally prepared, for example, by reacting the free acid or base forms of said compounds with a stoichiometric amount of the suitable base or acid in water or in an organic solvent or in a mixture of the two. Non-aqueous media such as ether, ethyl acetate, ethanol, acetone, isopropanol or acetonitrile are generally preferred. Illustrative examples of said acid addition salts include inorganic acid addition salts such as, for example, hydrochloride, hydrobromide, hydroiodide, sulfate, nitrate, phosphate, etc., organic acid addition salts such as, for example, acetate, maleate, fumarate, citrate, oxalate, succinate, tartrate, malate, mandelate, methanesulfonate, *p*-toluenesulfonate, trifluoroacetate, camphorsulfonate, etc. Illustrative examples of base addition salts include inorganic base salts such as, for example, ammonium salts and organic base salts such as, for example, ethylenediamine, ethanolamine, *N,N*-dialkylenethanolamine, triethanolamine, glutamine, amino acid basic salts, etc. Illustrative examples of metal salts include, for example, sodium, potassium, calcium, magnesium, aluminum and lithium salts.

The term "solvate" according to this invention is to be understood as meaning any form of the compound which has another molecule (most likely a polar solvent) attached to it via non-covalent bonding. Examples of solvate include hydrates and alcoholates, e.g. methanolates. Methods of solvation are generally known within the art. The compounds of the invention may present different polymorphic forms, and it is intended that the invention encompasses all such forms.

By "room temperature" or its abbreviation "rt" is meant herein that the reactions or processes are performed without heating or cooling. Generally, by room temperature may be understood as a temperature between about 15 °C and about 30 °C, or more particularly between about 20 °C and about 25 °C.

The term "organic solvent" includes for example cyclic and acyclic ethers (e.g. Et₂O, iPr₂O, tBu₂O, MeOtBu, 1,4-dioxane, tetrahydrofuran, methyltetrahydrofuran), hydrocarbon solvents (e.g. pentane, hexane, cyclohexane, heptane), halogenated solvents (e.g. dichloromethane, chloroform), alcohols (e.g. methanol, ethanol, propanol, isopropanol, sec-butanol, t-butanol), aromatic solvents (e.g. toluene, xylene), ketones (e.g. acetone, butanone, pentanone, methyl ethyl ketone, ethyl isopropyl ketone), esters (e.g. EtOAc, iPrOAc), nitriles (e.g. acetonitrile, benzonitrile, propionitrile), amides (e.g. DMF, DMA, HMPA), sulfoxides (DMSO) and mixtures thereof.

The term "vol" refers to volume equivalents, that is, the milliliters of solvent per gram reference starting material. For instance, 1 vol means 1 mL solvent per 1 g reference starting material.

### Process of the invention

In an aspect, the invention is directed to a process for preparing a compound of Formula (VIII) or a salt or solvate thereof from a compound of Formula (IV) or a salt or solvate thereof, said process comprising steps (a1), (b1), (c) or steps (a2), (b2), (c) as defined herein.

In some embodiments, R¹ is selected from the group consisting of C₁-C₆ alkyl, C₃-C₇ cycloalkyl, C₆-C₁₀ aryl, and (C₆-C₁₀)aryl(C₁-C₆)alkyl. Preferably, R¹ is C₁-C₆ alkyl such as methyl or ethyl or (C₆-C₁₀)aryl(C₁-C₆)alkyl such as benzyl.

In some embodiments, R² is C₁-C₆ alkyl such as methyl or ethyl.

In some embodiments, Y is N or CR³, wherein R³ is H or C₁-C₆ haloalkyl. Preferably, R³ is H or trifluoromethyl. More preferably, R³ is H.

In some embodiments, Z is N or CR⁴, wherein R⁴ is selected from the group consisting of H, cyano, C₁-C₆ haloalkoxy and C₁-C₆ haloalkyl, Preferably, R⁴ is selected from the group consisting of H, cyano, trifluoromethoxy and trifluoromethyl. More preferably, R⁴ is H.

In some embodiments, R⁵ is selected from the group consisting of is selected from the group consisting of H, C₁-C₆ alkyl, C₃-C₇ cycloalkyl, 3- to 10-membered heterocyclyl, C₁-C₆ alkoxy, C₁-C₆ haloalkoxy, C₁-C₆ haloalkyl, and halogen. In some embodiments, R⁵ is C₃-C₇ cycloalkyl. Preferably, R⁵ is selected from the group consisting of H, carboxamide, chloro, cyano, cyclobutyl, cyclohexyl, cyclopentyl, cyclopentyloxy, cyclopropyl, cyclopropylmethoxy, cyclohexylmethyl, 3,3-difluoropyrrolidin-1-yl, ethylamino, isobutyl, isopropoxy, methylsulfonyl, neopentyl, propyl, pyrrolidin-1-yl, 1,2,3-thiadiazol-4-yl, trifluoromethoxy and trifluoromethyl. More preferably, R⁵ is selected from the group consisting of H, chloro, cyclobutyl, cyclohexyl, cyclopentyl, cyclopropyl, 3,3-difluoropyrrolidin-1-yl, isobutyl, isopropoxy, neopentyl, propyl, pyrrolidin-1-yl, trifluoromethoxy and trifluoromethyl. Even more preferably, R⁵ is cyclopentyl.

In some embodiments, R⁶ is selected from the group consisting of H, cyano, C₁-C₆ haloalkoxy and C₁-C₆ haloalkyl. Preferably, R⁶ is selected from the group consisting of H, cyano, trifluoromethoxy and trifluoromethyl. More preferably, R⁶ is trifluoromethyl.

In preferred embodiments,
R¹ is C₁-C₆ alkyl, preferably methyl or ethyl, or (C₆-C₁₀)aryl(C₁-C₆)alkyl, preferably benzyl; more preferably, R¹ is C₁-C₆ alkyl, preferably methyl or ethyl;
R² is C₁-C₆ alkyl, preferably methyl or ethyl;
Y and Z are CH;
R⁵ is cyclopentyl; and
R⁶ is trifluoromethyl.

### Preparation of compounds of Formula (IV)

In some embodiments, the compound of Formula (IV) or a salt or solvate thereof is obtained by reduction of a compound of Formula (III) or a salt or solvate thereof: 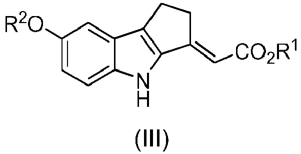 wherein
R¹ and R² are as defined for the compound of Formula (IV).

The reduction of a compound of Formula (III) is normally conducted in the presence of a reduction agent, a catalyst and a solvent.

In some embodiments, the reduction agent comprises formic acid and a base.

In some embodiments, the base is an inorganic base such as a carbonate base, e.g. sodium carbonate, potassium carbonate, or cesium carbonate.

In some embodiments, the base is an organic base such as an amine, e.g. ammonia, dimethylamine, diethylamine, trimethylamine, or triethylamine. In some embodiments, the base is trimethylamine.

In some embodiments, the catalyst comprises palladium such as palladium on carbon. In some embodiments, the catalyst is palladium on carbon (Pd/C), from about 2% to about 10% palladium. In some embodiments, the catalyst is about 10% palladium on carbon.

In some embodiments, the solvent is selected from the group consisting of ethyl acetate, tetrahydrofuran and acetone. In some embodiments, the solvent is tetrahydrofuran or ethyl acetate.

In some embodiments, the reduction of a compound of Formula (III) is conducted under a substantially inert atmosphere such as a substantially inert atmosphere comprising argon or nitrogen.

In some embodiments, the molar ratio between the compound of Formula (III), formic acid, and the base is about 1.0:1.0:1.0 to about 1.0:6.0:6.0, more particularly about 1.0:1.0:1.0 to about 1.0:5.0:5.0, more particularly about 1.0:2.0:2.0 to about 1.0:4.0:4.0, more particularly about 1.0:2.0:2.0 to about 1.0:3.0:3.0, more particularly about 1.0:3.0:3.0.

In some embodiments, the amount of 10% palladium on carbon is about 0.1 g to about 0.3 g per g of compound of Formula (III), e.g. about 0.2 g per g of compound of Formula (III).

In some embodiments, the amount of solvent, e.g. tetrahydrofuran or ethyl acetate, is about 15 ml to about 25 ml per g of compound of Formula (III), e.g. about 20 ml per g of compound of Formula (III).

In some embodiments, the reduction of a compound of Formula (III) is conducted at a temperature of about 15 °C to about 55 °C, more particularly about 20 °C to about 45 °C, more particularly about 25 °C to about 35 °C, more particularly about 30 °C.

In some embodiments, the reduction of a compound of Formula (III) is conducted by adding formic acid to a mixture comprising the compound of Formula (III), the catalyst, the base and the solvent.

In some embodiments, the reduction of a compound of Formula (III) is conducted by adding formic acid to a mixture comprising the compound of Formula (III), Pd/C 10%, trimethylamine and tetrahydrofuran or ethyl acetate and carrying out the reaction at a temperature of about 25 °C to about 35 °C, wherein the compound of Formula (III) and the compound of Formula (IV) are, respectively:

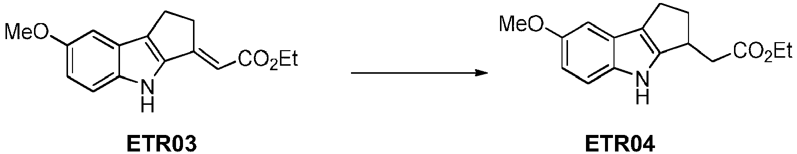

In some embodiments, the compound of Formula (IV) is isolated. In some embodiments, the compound of Formula (IV) is further transformed without isolation in a one-pot fashion.

### Preparation of compounds of Formula (III)

In some embodiments, the compound of Formula (III) or a salt or solvate thereof is obtained by reaction of a compound of Formula (I) or a salt or solvate thereof 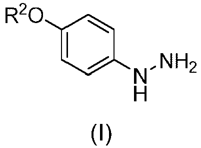 wherein
R² is as defined for the compound of Formula (IV);
with a compound of Formula (II) or a salt or solvate thereof 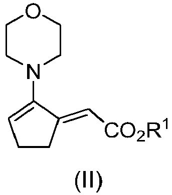
wherein
R¹ is as defined for the compound of Formula (VIII).

In some embodiments, the compounds of Formula (I), (II) and (III) are, respectively, as follows:

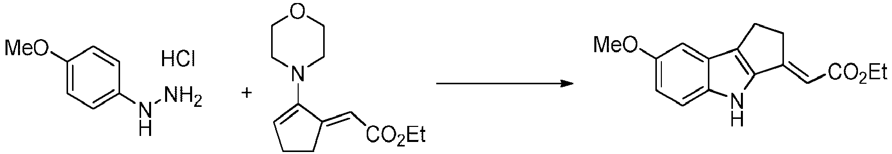

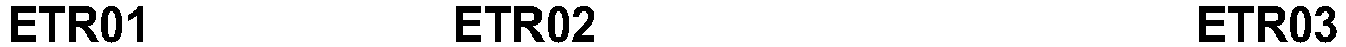

In some embodiments, the reaction between the compound of Formula (I) with the compound of Formula (II) is conducted in the presence of an acid and a solvent.

In some embodiments, such acid is a mixture of acetic and trifluoroacetic acid. In some embodiments, such acid is trifluoroacetic acid.

In some embodiments, the solvent in the reaction between the compound of Formula (I) with the compound of Formula (II) is selected from the group consisting of methanol and ethanol. In some embodiments, the solvent is ethanol.

In some embodiments, the molar ratio between the compound of Formula (I) and the compound of Formula (II) is about 1.0:1.0 to about 1.0:1.5, more particularly about 1.0:1.0 or 1.0:1.1.

In some embodiments, the amount of acid ranges from about 1 to about 3 vol, more particularly from about 1.5 to about 2.5 vol. In some embodiments, the acid is a mixture of acetic acid about 1.75 vol and trifluoroacetic acid about 0.5 vol. In some embodiments, the acid is trifluoroacetic acid about 2.2 vol.

In some embodiments, the reaction between the compound of Formula (I) with the compound of Formula (II) is conducted at a temperature of about 40 °C to about 80 °C, more particularly about 50 °C to about 70 °C, more particularly about 60 °C to about 65 °C.

### Preparation of compounds of Formula (V) and compounds of Formula (VII) by enantioselective enzymatic hydrolysis

The compound of Formula (V) or a salt or solvate thereof is obtained by enantioselective enzymatic hydrolysis of a compound of Formula (IV) or a salt or solvate thereof in the presence of a lipase selected from *Thermomyces lanuginosus* lipase and *Candida antarctica* lipase B.

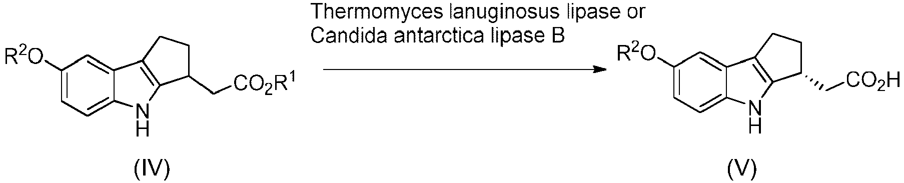

In some embodiments, R¹ is Et and R² is Me in the compound of Formula (IV) (**ETR04**) and R² is Me in the compound of Formula (V) (**ETR05**).

Likewise, the compound of Formula (VII) or a salt or solvate thereof is obtained by enantioselective enzymatic hydrolysis of a compound of Formula (XII) or a salt or solvate thereof in the presence of a lipase selected from *Thermomyces lanuginosus* lipase and *Candida antarctica* lipase B.

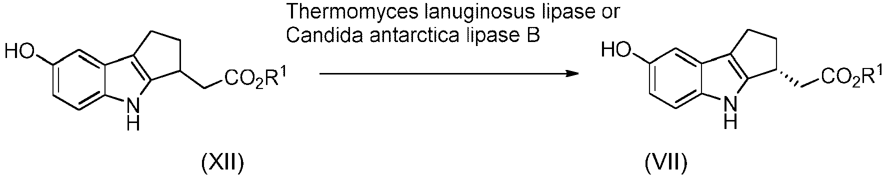

In some embodiments, R¹ is Me or Et in the compound of Formula (XII) (**ETR12-Me, ETR12-Et**) and R¹ is Me or Et in the compound of Formula (VII) (**ETR07-Me**, **ETR07-Et**).

In some embodiments, the compound of Formula (IV) is obtained and without isolation in a one-pot fashion is subjected to enantioselective enzymatic hydrolysis.

According to the present invention, a lipase selected from *Thermomyces lanuginosus* lipase (TLL) and *Candida antarctica* lipase B (CALB) is used as catalyst for the enantioselective hydrolysis, more preferably *Thermomyces lanuginosus* lipase (TLL). The lipase from *Thermomyces laguginosus* (formerly *Humicola laguginosa*) and the lipase B from *Candida antarctica* are commercially available in both soluble and immobilized form.

In some embodiments, *Thermomyces lanuginosus* lipase is produced by a submerged fermentation of *Aspergillus sp.* (commonly referred to as native *Aspergillus sp.* Lipase). In some embodiments, *Candida antarctica* lipase B is selected from *Candida antarctica* lipase B recombinant from yeast and *Candida antarctica* lipase B recombinant from *Aspergillus oryzae.*

In some embodiments, the lipase is immobilized. The term immobilized designates that the lipase is either physically or chemically confined or localized in a certain space or region with a plenteous holding of its catalytic activity in several cycles to follow. The immobilization of enzymes can enhance enzyme stability and catalytic power.

In some embodiments, the lipase is immobilized native *Aspergillus sp.* lipase (e.g. lipase commercialized as NATE-1753 by Creative Enzymes^{®}) or immobilized *Candida antarctica* lipase B, e.g *Candida antarctica* lipase B immobilized on Immobead 150. In some embodiments, the lipase is immobilized *Candida antarctica* lipase B recombinant from yeast (e.g. lipase commercialized as Lipase B *Candida antarctica* immobilized on Immobead 150, recombinant from yeast, by Sigma-Aldrich with catalogue number 52583). In some embodiments, the lipase is immobilized *Candida antarctica* lipase B recombinant *Aspergillus oryzae* (e.g. lipase commercialized as Lipase B Candida antarctica immobilized on Immobead 150, recombinant from *Aspergillus oryzae,* by Sigma-Aldrich with catalogue number 54326).

Preferably, the lipase is *Thermomyces lanuginosus* lipase (TLL), and more preferably immobilized native *Aspergillus sp.* Lipase.

Other lipases such as Lipase A *Candida Antarctica* immobilized on Immobead 150, recombinant from *Aspergillus oryzae* does not catalyze the enantioselective hydrolysis (see example 4).

In some embodiments, the weight ratio between the compound of Formula (IV) or (XII) and the lipase is from about 15.0:1.0 to about 5.0:1.0, more particularly from about 15.0:1.0 to about 10.0:1.0.

The enantioselective enzymatic hydrolysis is normally conduced in the presence of a solvent. In some embodiments, the solvent is dimethylformamide (DMF), dimethylacetamide (DMA), dimethylsulfoxide (DMSO), tetrahydrofuran (THF), or acetonitrile. In some embodiments, the solvent is acetonitrile.

In some embodiments, the amount of solvent ranges from about 5 to about 20 vol, more particularly from about 10 to about 15 vol.

In some embodiments, the enantioselective enzymatic hydrolysis is conducted in the presence of a buffer, preferably a phosphate buffer. In some embodiments, the enantioselective enzymatic hydrolysis is conducted in the presence of a phosphate buffer at a pH of about 6.0 to about 9.0. In some embodiments, the enantioselective enzymatic hydrolysis is conducted in the presence of a phosphate buffer at a pH of about 7.0 to about 8.0. In some embodiments, the enantioselective enzymatic hydrolysis is conducted in the presence of a phosphate buffer at a pH of about 7.3 to about 7.8. In some embodiments, the enantioselective enzymatic hydrolysis is conducted in the presence of a phosphate buffer at a pH of about 7.5. In some embodiments, the phosphate buffer is a sodium phosphate buffer. In some embodiments, the phosphate buffer is a potassium phosphate buffer.

In some embodiments, the enantioselective enzymatic hydrolysis is conducted at a temperature of about 0 °C to about 75 °C. In some embodiments, the enantioselective enzymatic hydrolysis is conducted at a temperature of about 20 °C to about 65 °C. In some embodiments, the enantioselective enzymatic hydrolysis is conducted at a temperature of about 30 °C to about 55 °C. In some embodiments, the enantioselective enzymatic hydrolysis is conducted at a temperature of about 40 °C to about 45 °C.

Since the enzyme reacts with only one of the two enantiomers, the enantioselective hydrolysis of a racemic mixture can only result in a 50% yield at best. In some embodiments, the undesired (S)-isomer ester of Formula (IV) or (XII) that remains non-hydrolyzed may be epimerized and subjected to a further enantioselective hydrolysis. This operation allows increasing the yield of this step above 50% (example 7 for instance reports a yield of 59%).

In some embodiments, the compound obtained after the enantioselective enzymatic hydrolysis has an enantiomeric excess of about 95% or greater, such as about 96% or greater, about 97% or greater, about 98% or greater or about 99% or greater.

### Preparation of compounds of Formula (VI) and/or (VI-BBr₃) and compounds of Formula (XII) by hydroxyl deprotection

The compound of Formula (VI-BBr₃) and/or (VI) or a salt or solvate thereof is obtained by hydroxyl deprotection of a compound of Formula (V) or a salt or solvate thereof, using boron tribromide as hydroxyl deprotection agent followed by quenching with a tertiary alcohol.

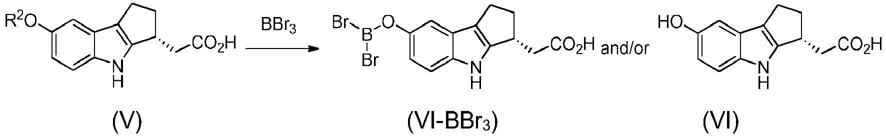

In some embodiments, R² is Me in the compound of Formula (V) (**ETR05**).

The compound of Formula (XII) or a salt or solvate thereof is obtained by hydroxyl deprotection of a compound of Formula (IV) or a salt or solvate thereof, using any hydroxyl deprotection agent.

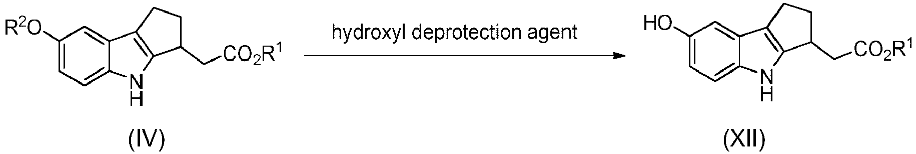

In some embodiments, R¹ is Et and R² is Me in the compound of Formula (IV) (**ETR04**) and R¹ is Me or Et in the compound of Formula (XII) (**ETR12-Me**, **ETR12-Et**)**.**

Any hydroxyl deprotection agent such as boron tribromide or methionine may be used for the transformation of the compound of Formula (IV) or a salt or solvate thereof into a compound of Formula (XII) or a salt or solvate thereof since they are racemic compounds. The selection of appropriate hydroxyl deprotection agents can be readily determined by one skilled in the art. However, when the starting material is the compound of Formula (V) or a salt or solvate thereof, it has been found that the specific use of boron tribromide as hydroxyl deprotection agent and subsequent quenching with a tertiary alcohol (such as t-butanol) is necessary in order to retain the enantioselectivity introduced in the previous enzymatic hydrolysis. These conditions allow, if desired, isolating and purifying in an efficient way the intermediate of Formula (VI-BBr₃):

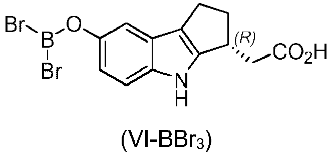

Alternatively, the compound of Formula (VI-BBr₃) is not isolated but the alcohol compound of Formula (VI). Moreover, the alcohol compound of Formula (VI) may be also subsequently esterificated without isolation. Thus, it is possible to transform a compound of Formula (V) into a compound of Formula (VII) in a one-pot fashion.

Besides the hydroxyl deprotection agent, the hydroxyl deprotection of a compound of Formula (V) or Formula (IV) is normally conducted in the presence of a solvent.

In some embodiments, the solvent is dichloromethane. In some embodiments, the amount of solvent ranges from about 5 to about 20 vol, more particularly from about 5 vol to about 35 vol.

In some embodiments, the hydroxyl deprotection of the compound of Formula (V) or Formula (IV) is conducted under a substantially inert atmosphere such as a substantially inert atmosphere comprising argon or nitrogen.

In some embodiments, the molar ratio between the compound of Formula (V) or Formula (IV) and the hydroxyl deprotection agent is about 1.0:1.0 to about 1.0:5.0, more particularly form about 1.0:2.0 to about 1.0:4.0, more particularly about 1.0:3.0.

In some embodiments, the hydroxyl deprotection of a compound of Formula (V) or Formula (IV) is conducted at a temperature of about 0 °C to about 10 °C, more particularly about 0 °C to about 5 °C.

In some embodiments, the hydroxyl deprotection of a compound of Formula (V) or Formula (IV) is conducted by adding boron tribromide to the compound of Formula (V) or Formula (IV) and dichloromethane and carrying out the reaction at a temperature of about 0 °C to about 5 °C.

The tertiary alcohol used for quenching the may be selected for instance from the group consisting of t-butyl alcohol, t-amyl alcohol, t-hexyl alcohols, t-heptyl alcohols and the t-octyl alcohols. Preferably, the tertiary alcohol is t-butyl alcohol (t-butanol).

It has been found that the use of other quenching agents such as NaOHaq or water results in slight or substantial racemization (see examples 9 and 10, respectively).

The compound of Formula (VI-BBr₃), as previously described, may be efficiently isolated and purified but it may be also further transformed without isolation in a one-pot fashion into an alcohol compound of Formula (VI) or even an ester compound Formula (VII). In some embodiments, the compound of Formula (VI) is isolated. However, the compound of Formula (VI) is preferably further transformed without isolation in a one-pot fashion into a compound of Formula (VII). The compound of Formula (XII) may be isolated or further transformed without isolation in a one-pot fashion into a compound of Formula (VII).

### Preparation of compounds of Formula (VII): Esterification of the compounds of Formula (VI) or Formula (VI-BBr₃)

The compound of Formula (VII) or a salt or solvate thereof is obtained by esterification in basic media of a compound of Formula (VI) and/or Formula (VI-BBr₃), or a salt or solvate thereof.

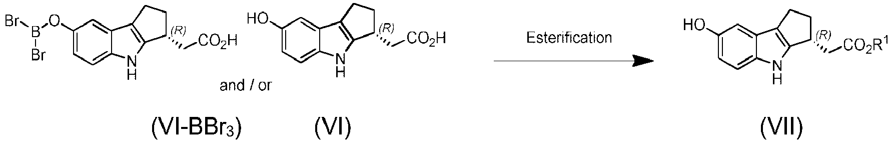

In some embodiments, the compound of Formula (VI) and/or (VI-BBr₃) is obtained and without isolation in a one-pot fashion is subjected to esterification.

The esterification of a compound of Formula (VI) and/or (VI-BBr₃) into a compound of Formula (VII) in basic media is conducted in the presence of a base (i.e. basic esterification). In some embodiments, the base comprises an inorganic base such as a carbonate base, e.g. sodium carbonate, potassium carbonate, or cesium carbonate. In a more particular embodiment, the base is potassium carbonate.

It has been found that the esterification in acid media results in racemization (see example 11).

The esterification of a compound of Formula (VI) and/or (VI-BBr₃) into a compound of Formula (VII) is also normally conducted in the presence of an esterification agent and a suitable solvent.

In some embodiments, the esterification agent comprises a C₁-C₆ alkyl halide, e.g. methyl iodide or ethyl iodide (resulting respectively in **ETR07-Me** and **ETR07-Et**) or a (C₆-C₁₀)aryl(C₁-C₆)alkyl halide, e.g. benzyl bromide (resulting in **ETR07-Bn**).

In some embodiments, the solvent is dimethylformamide (DMF).

In some embodiments, the molar ratio between the compound of Formula (VI)/(VI-BBr₃), esterification agent, and esterification base is about 1.0:1.0:1.0 to about 1.0:2.0:2.0, more particularly about 1.0:1.0:1.0 to about 1.0:1.5:1.5, more particularly about 1.0:1.0:1.0 to about 1.0:1.2:1.2, more particularly about 1.0:1.0:1.0 to about 1.0:1.1:1.1, more particularly about 1.0:1.1:1.0.

In some embodiments, the esterification of a compound of Formula (VI)/(VI-BBr₃) is conducted at a temperature of about 30 °C to about 70 °C, more particularly about 40 °C to about 60 °C, more particularly about 45 °C to about 55 °C, more particularly about 50 °C.

### Preparation of compounds of Formula (VIII)

The compound of Formula (VIII) or a salt or solvate thereof is obtained by reaction of a compound of Formula (VII) or a salt or solvate thereof and a compound of Formula (XI) or a salt or solvate thereof:

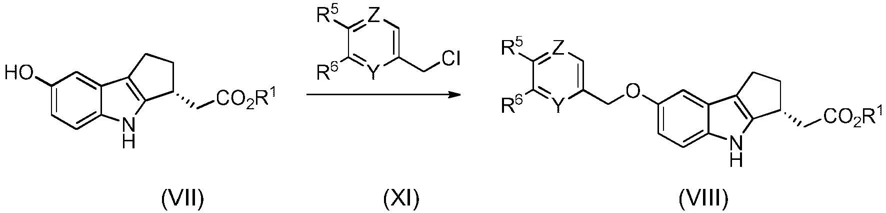

In some embodiments, R¹ is Me, Et or Bn in the compound of Formula (VII) (**ETR07-Me, ETR07-Et, ETR07-Bn**), Y and Z are CH, R⁵ is cyclopentyl and R⁶ is trifluoromethyl in the compound of Formula (XI) (**ETR11**) and R¹ is Me or Et in the compound of Formula (VIII) (**ETR08-Me**, **ETR08-Et**, **ETR08-Bn**).

The reaction of a compound of Formula (VII) and a compound of Formula (XI) is normally conducted in the presence of a base and a solvent.

In some embodiments, the base is an inorganic base such as a carbonate base, e.g. sodium carbonate, potassium carbonate, or cesium carbonate. In some embodiments, the base is cesium carbonate.

In some embodiments, the solvent is selected from the group consisting of acetone and acetonitrile.

In some embodiments, the reaction of a compound of Formula (VII) and a compound of Formula (XI) is conducted under a substantially inert atmosphere such as a substantially inert atmosphere comprising argon or nitrogen.

In some embodiments, the molar ratio between the compound of Formula (VII), the compound of Formula (IX) and the base is about 1.0:1.0:1.0 to about 1.0:2.0:2.0, more particularly about 1.0:1.0:1.0 to about 1.0:1.5:1.5, more particularly about 1.0:1.0:1.0 to about 1.0:1.4:1.4, more particularly about 1.0:1.0:1.0 to about 1.0:1.2:1.2, more particularly about 1.0:1.0:1.0 to about 1.0:1.1:1.1, more particularly about 1.0:1.1:1.0.

In some embodiments, the reaction of a compound of Formula (VII) and a compound of Formula (XI) is conducted at about the reflux temperature of the solvent.

In some embodiments, the reaction of a compound of Formula (VII) and a compound of Formula (XI) is conducted in the presence of acetonitrile, preferably at a temperature of about 30 °C to about 70 °C, more particularly about 40 °C to about 60 °C, more particularly about 45 °C to about 55 °C, more particularly about 50 °C.

In some embodiments, the reaction of a compound of Formula (VII) and a compound of Formula (XI) is conducted by adding the compound of Formula (XI) to a mixture comprising the compound of Formula (VII), the base and the solvent.

In some embodiments, the reaction of a compound of Formula (VII) and a compound of Formula (XI) is conducted by adding the compound of Formula (XI) to a mixture comprising the compound of Formula (VII), cesium carbonate and the solvent (acetone or acetonitrile) and carrying out the reaction at about the reflux temperature of the solvent.

In some embodiments, the compound of Formula (VIII) is isolated. In some embodiments, the compound of Formula (VIII) is further transformed without isolation in a one-pot fashion.

Preferably, the compound of Formula (VIII) is isolated and crystallized. If desired, crystallization may be favored by adding seed crystals.

### Preparation of compounds of Formula (IX): Etrasimod and related compounds

The compound of Formula (IX) or a salt or solvate thereof can be obtained by hydrolysis of a compound of Formula (VIII) or a salt or solvate thereof, as depicted in the following scheme:

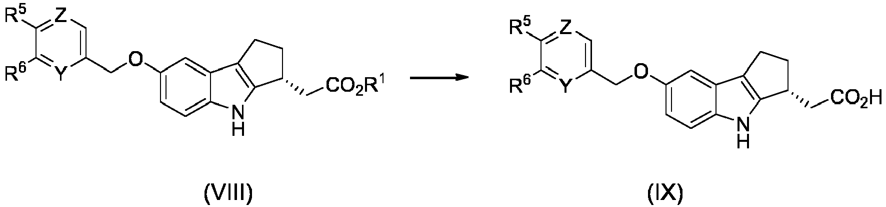

In some embodiments, the hydrolysis is a basic hydrolysis, an enzymatic hydrolysis or a hydrogenolysis. Basic and enzymatic hydrolysis is particularly preferred when R¹ is C₁-C₆ alkyl such as methyl or ethyl or C₃-C₇ cycloalkyl.

The basic hydrolysis of a compound of Formula (VIII) is normally conducted in the presence of a base and a solvent.

In some embodiments, the base is an inorganic base such as a hydroxide, e.g. lithium or sodium hydroxide. In some embodiments, the base is lithium hydroxide.

In some embodiments, the solvent in the basic hydrolysis is selected from the group consisting of dioxane, ethanol and THF. In some embodiments, the solvent is dioxane.

In some embodiments, the basic hydrolysis of a compound of Formula (VIII) is conducted under a substantially inert atmosphere such as a substantially inert atmosphere comprising argon or nitrogen.

In some embodiments, the molar ratio between the compound of Formula (VIII) and the base is about 1.0:1.0 to about 1.0:6.0, more particularly about 1.0:1.0 to about 1.0:5.0, more particularly about 1.0:1.0 to about 1.0:5.0, more particularly about 1.0:1.0 to about 1.0:4.0 , more particularly about 1.0:3.0.

In some embodiments, the hydrolysis of a compound of Formula (VIII) is conducted at a temperature of about 15 °C to about 40 °C, more particularly about 20 °C to about 25 °C.

In some embodiments, the hydrolysis of a compound of Formula (VIII) is conducted by adding the base to the compound of Formula (VIII) in the solvent.

In some embodiments, the hydrolysis of a compound of Formula (VIII) is conducted by adding lithium hydroxide to the compound of Formula (VIII) in a solvent and carrying out the reaction at a temperature of about 20 °C to about 25 °C.

The enzymatic hydrolysis of a compound of Formula (VIII) is normally conducted in the presence of a lipase and a solvent. Lipases, solvents and any other conditions above described for the enantioselective enzymatic hydrolysis of a compound of Formula (IV) or (XII) are equally applicable to the enzymatic hydrolysis of a compound of Formula (VIII).

The compound of Formula (IX) or a salt or solvate thereof can be also obtained by hydrogenolysis of a compound of Formula (VIII) or a salt or solvate thereof, particularly when R¹ is (C₆-C₁₀)aryl such as phenyl or (C₆-C₁₀)aryl(C₁-C₆)alkyl such as benzyl. The hydrolysis with hydrogen is normally conducted in the presence of a catalyst (e.g. a palladium catalyst such as Pd/C).

In some embodiments, the compound of Formula (IX) is isolated. In some embodiments, the compound of Formula (IX) is further transformed, e.g. into a salt thereof, without isolation in a one-pot fashion.

In some embodiments, the compound of Formula (IX) is (R)-2-(7-(4-cyclopentyl-3-(trifluoromethyl)benzyloxy)-1,2,3,4-tetrahydrocyclopenta[b]indol-3-yl)acetic acid, i.e. etrasimod (**ETR09**).

The L-arginine salt of etrasimod (**ETR10**) may be prepared reacting etrasimod with L-arginine.

In some embodiments, etrasimod L-arginine is obtained by adding L-arginine in water to a salt-forming mixture comprising etrasimod, and a water-miscible anti-solvent, and heating.

The phrase "water-miscible anti-solvent" as used herein refers to a water soluble solvent in which a product, such as the L-arginine salt of (*R*)-2-(7-(4-cyclopentyl-3-(trifluoromethyl)-benzyloxy)-1,2,3,4-tetrahydrocyclopenta[b]indol-3-yl)acetic acid, has limited solubility. In some embodiments, the water-miscible anti-solvent is selected from the group consisting of acetonitrile, methanol, isopropanol, acetone or a mixture thereof. In some embodiments, the water-miscible anti-solvent is acetonitrile.

By proceeding in this way, etrasimod L-arginine may be obtained in crystal form.

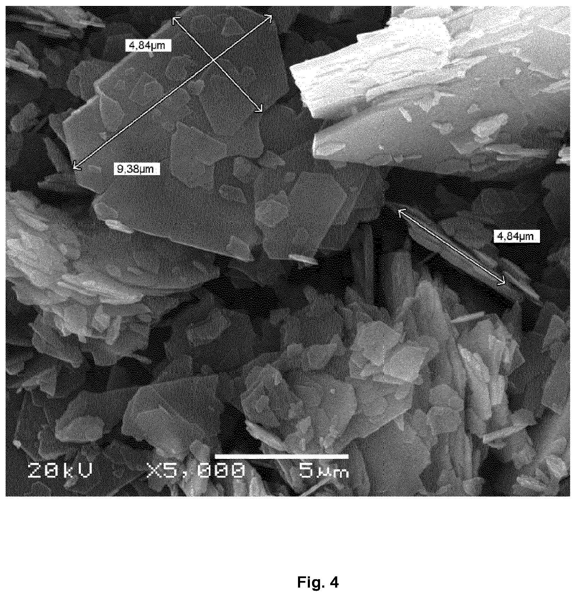

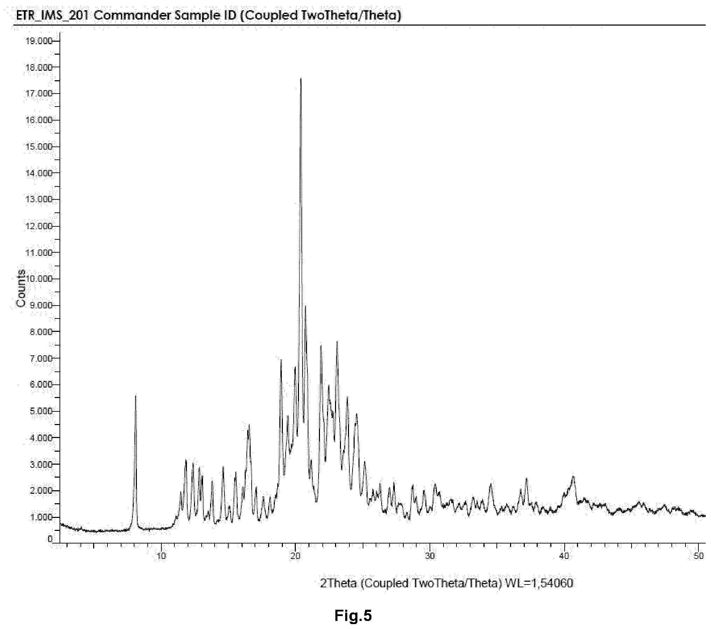

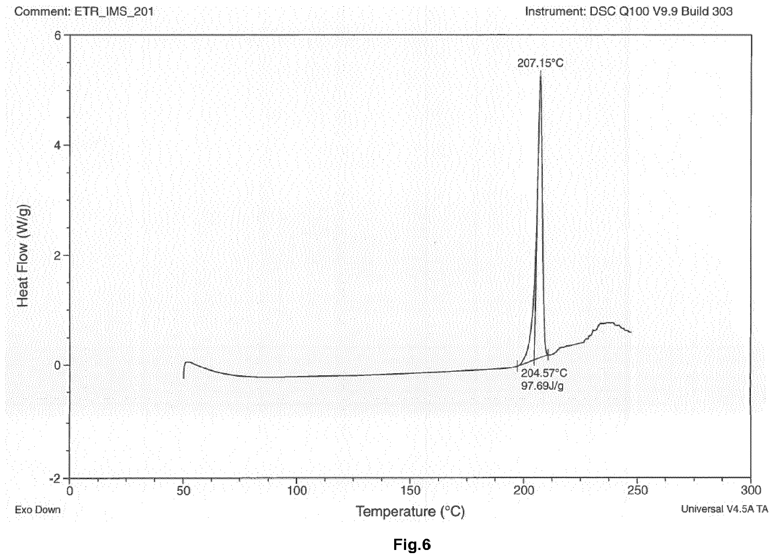

In an embodiment, the crystalline etrasimod arginine salt is characterized by having a SEM imagen substantially as depicted in figure 4 and/or a PXRD pattern substantially as depicted in figure 5 and/or a DSC analysis substantially as depicted in figure 6.

In an embodiment, amorphous etrasimod L-arginine is obtained by a process comprising:
adding acetonitrile to a suspension of etrasimod L-arginine in water;
stirring the mixture until completely dissolved;
filtering the solution;
freeze-drying (lyophilizating) the solution.

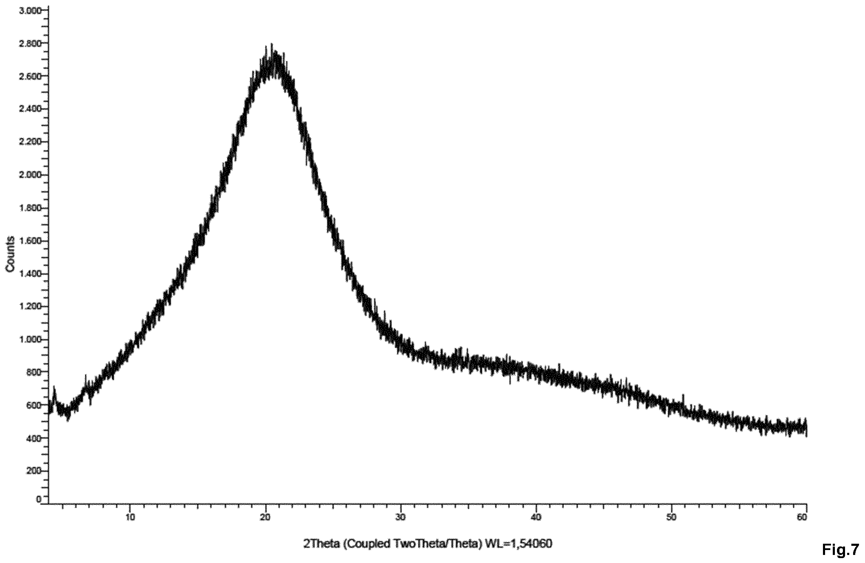

In an embodiment, the amorphous etrasimod arginine salt is characterized by having a powder X-ray diffraction (PXRD) pattern comprising a characteristic diffraction peak at 2-theta (2θ) values of about 20.5°; particularly, wherein its PXRD pattern comprises further characteristic diffraction peaks at 2θ values of about 4.4° and 6.7°; more particularly, wherein its PXRD pattern is substantially as depicted in figure 7.

### Intermediates of the invention

In a further aspect, the invention is directed to:

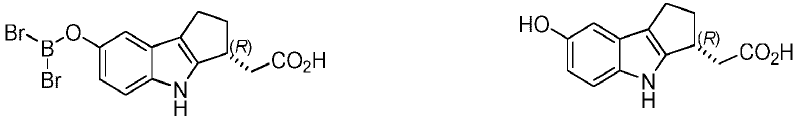

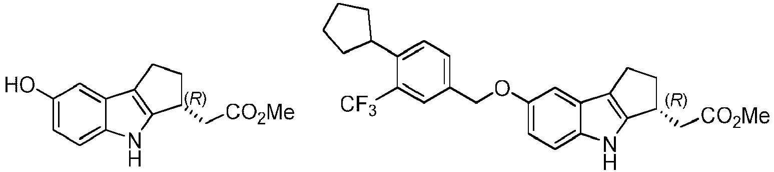

The following examples are merely illustrative of certain embodiments of the invention and cannot be considered as restricting it in any way.

### EXAMPLES

### Example 1. Preparation of ethyl 2-(7-methoxy-1,4-dihydrocyclopenta[b]indol-3(2H)-ylidene)acetate (ETR03).

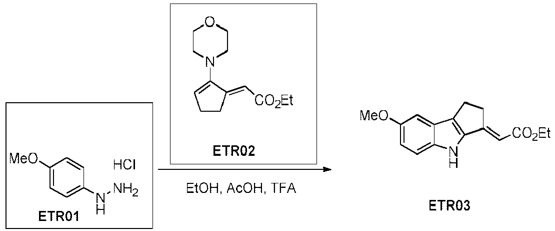

To a 1 L round-bottom flask, (4-methoxyphenyl)hydrazine hydrochloride (15 g, 86 mmol), ethyl 2-(2-morpholinocyclopent-2-enylidene)acetate (20.4 g, 86 mmol), EtOH (225 mL, 15 vol), and trifluoroacetic acid (33 mL, 2.2 vol, a bit exothermic reaction) were added. The mixture was stirred at 40°C for 0.5 h and the mixture was maintained at 60/65°C overnight. The reaction mixture was cooled and aqueous solution of 30% NaOH (16.5 ml) was added until a pH about pH 4.8, and a suspension was formed. The suspension was stirred at 0-5°C for 1h and then filtered. The filter cake was washed with cold EtOH (45 mL, 3 vol), water (300 mL, 20 vol) and cold EtOH (45 mL, 3 vol). The solid was dried at 40°C under vacuum for 12 hours affording the title compound (11.2 g).
UPLC-MS m/z=272 [M+H]+.
¹H NMR (500 MHz, CDCl₃) δ 10.30 (s, 1H), 7.30 (d, J = 8.7 Hz, 1H), 7.00 - 6.93 (m, 2H), 5.64 (m, 1H), 4.23 (q, J = 7.1 Hz, 2H), 3.86 (s, 3H), 3.27 (m, 2H), 2.97 (m, 2H), 1.34 (t, J = 7.1 Hz, 3H).

### Ratio of isomers UPLC (E/Z): 92:8.

### Example 2. Preparation of ethyl 2-(7-methoxy-1,2,3,4-tetrahydrocyclopenta[b]indol-3-yl)acetate (ETR04).

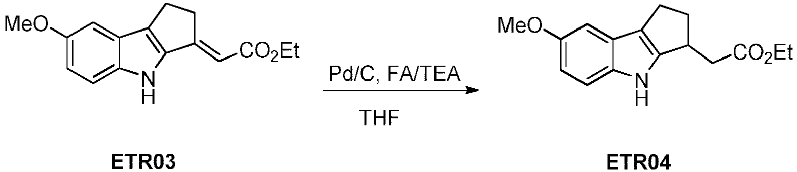

To a 250 mL round-bottom flask, ethyl 2-(7-methoxy-1,4-dihydrocyclopenta[b]indol-3(2H)-ylidene)acetate (9.1 g, 33.57 mmol) and THF (72.8 mL, 8 vol) were added and the resulting mixture was stirred under nitrogen for 5 minutes. Then, 10% Pd/C (50% wet, 3.64 g) and NEt₃ (23.39 mL, 167.83 mmol) were added maintaining the temperature below 30°C. To the resulting mixture formic acid (6.33 mL, 167.83 mmol) was added dropwise maintaining the temperature below 30°C and the reaction was stirred at that temperature for 2h. The mixture was filtered through celite, washed with THF (2×20 mL) and the filtrate was removed under pressure at 40°C. To the resulting crude, AcOEt (5 mL) and water (50 mL) were added and the mixture was transferred to a separatory funnel. After phase separation was completed, the lower aqueous layer was drained. The organic solvent was removed under pressure at 40°C and purified by chromatography column affording the title compound as a yellowish oil (8.4 g).
UPLC-MS m/z=274 [M+H]+.
¹H NMR (500 MHz, CDCl₃) δ 8.45 (s, 1H), 7.20 (dd, *J* = 8.7, 1.1 Hz, 1H), 6.93 (m, 1H), 6.78 (dt, *J* = 8.8, 2.1 Hz, 1H), 4.22 (qdd, *J* = 7.1, 3.2, 1.4 Hz, 2H), 3.85 (d, *J* = 1.6 Hz, 3H), 3.56 (m, 1H), 2.90-2.70 (m, 4H), 2.52 (ddd, *J* = 16.7, 11.1, 1.3 Hz, 1H), 2.17 - 2.04 (m, 1H), 1.31 (td, *J* = 7.2, 1.4 Hz, 3H).

### Example 3. Preparation of (R)-2-(7-methoxy-1,2,3,4-tetrahydrocyclopenta[b]indol-3-yl)acetic acid (ETR05) with Lipase B Candida antarctica immobilized on Immobead 150, recombinant from Aspergillus oryzae.

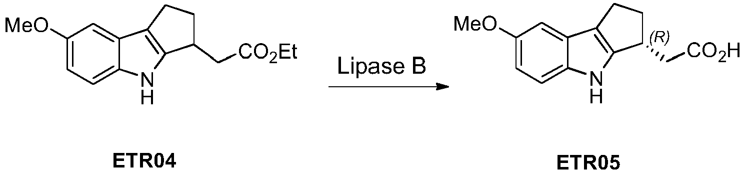

**Preparation of 1.0 M buffer solution:** A solution of 1.16 g of potassium phosphate monobasic in purified water (8.52 mL) was added to a solution of 14.7 g of potassium phosphate dibasic in purified water (84 mL) and was stirred at room temperature for 5 minutes.

To a 50 mL round-bottom flask, ethyl 2-(7-methoxy-1,2,3,4-tetrahydrocyclopenta[b]indol-3-yl)acetate (500 mg, 1.83 mmol), acetonitrile (7.5 mL, 15 vol), and a portion of 1.0 M buffer previously prepared (0.4 mL, 0.8 vol) were added and the mixture was stirred at room temperature for 5 minutes. The resulting mixture was stirred (mechanic agitation, 160 rpm) under nitrogen and Lipase B, *Candida antarctica* immobilized on Immobead 150, recombinant from *Aspergillus oryzae* (50 mg) was added and the reaction was stirred at 40 °C overnight. The mixture was cooled at room temperature and filtered to remove immobilized lipase and phosphate and citrate salts. The UPLC analysis showed a 46% of the desired compound and 54% of (S)-ester.
UPLC-MS m/z=246 [M+H]+.
Quiral HPLC: 98.70%

### Example 4 (Comparative). Preparation of (R)-2-(7-methoxy-1,2,3,4-tetrahydrocyclopenta[b]indol-3-yl)acetic acid (ETR05) with Lipase A Candida antarctica immobilized on Immobead 150, recombinant from Aspergillus oryzae.

Example 3 was reproduced except that the lipase was Lipase A *Candida antarctica* immobilized on Immobead 150, recombinant from *Aspergillus oryzae.* The reaction did not proceed, and only starting material was recovered.

### Example 5. One-pot preparation of (R)-2-(7-methoxy-1,2,3,4-tetrahydrocyclopenta[b]indol-3-yl)acetic acid (ETR05) from ethyl 2-(7-methoxy-1,4-dihydrocyclopenta[b]indol-3(2H)-ylidene)acetate (ETR03).

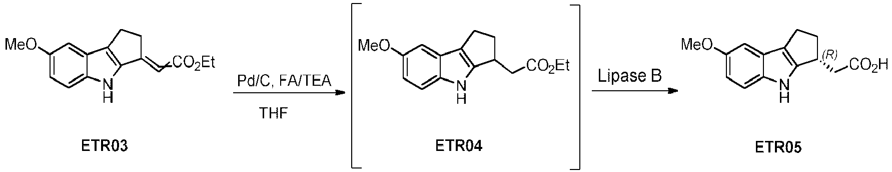

To a 500 mL round-bottom flask, ethyl 2-(7-methoxy-1,4-dihydrocyclopenta[b]indol-3(2H)-ylidene)acetate (20 g, 73.77 mmol) and THF (400 mL, 20 vol) were added and the resulting mixture was stirred under nitrogen for 5 minutes. Then, 10% Pd/C (50% wet, 0.2 g/g, 4 g) and NEt₃ (30.85 mL, 221.1 mmol, 3 eq.) were added maintaining the temperature below 30°C. To the resulting mixture, formic acid (8.34 mL, 221.1 mmol, 3 eq.) was added dropwise maintaining the temperature below 30°C and the reaction was stirred at that temperature for 1h. The mixture was filtered through celite, washed with THF (2×20 mL) and the filtrate was removed under pressure at 40°C. To the resulting crude, AcOEt (140 mL, 7 vol) and water (140 mL, 7 vol) were added and the mixture was transferred to a separatory funnel. After phase separation was completed, the lower aqueous layer was drained. The organic phase was washed with water (140 mL, 7 vol) and the organic solvent was removed under pressure at 40°C affording ethyl 2-(7-methoxy-1,2,3,4-tetrahydrocyclopenta[b]indol-3-yl)acetate (22 g, crude).

**Preparation of 1.0 M buffer solution:** A solution of 1.16 g of potassium phosphate monobasic in purified water (8.52 mL) was added to a solution of 14.7 g of potassium phosphate dibasic in purified water (84 mL) and was stirred at room temperature for 5 minutes.

To a 500 mL round-bottom flask with ethyl 2-(7-methoxy-1,2,3,4-tetrahydrocyclopenta[b]indol-3-yl)acetate (22 g) previously obtained, acetonitrile (200 mL, 10 vol) and a portion of 1.0 M buffer previously prepared (16 mL, 0.8 vol) were added and the mixture was stirred at room temperature for 5 minutes. The resulting mixture was stirred (mechanic agitation, 160 rpm) under nitrogen and Lipase B, *Candida Antarctica*, immobilized (1.6 g, 0,08g/g) was added and the reaction was stirred at 40 °C overnight. The mixture was cooled at room temperature and contents were filtered to remove immobilized lipase. The round-bottom flask and solids were washed with acetonitrile (3×20 mL) and the combined filtrates were concentrated under pressure. To the crude, ethyl acetate (140 mL, 7 vol), NaHCO₃ (7% aqueous solution, 140 mL, 7 vol) and water (60 mL, 3 vol) were added and transferred to a separatory funnel. After phase separation was completed, the organic phase was extracted with a solution of NaHCOa (7% aqueous solution, 60 mL, 3 vol).

DCM (140 mL, 7 vol) was added to the reunited aqueous phases and the pH was adjusted between 4 and 5 by addition of HCl (18% aqueous solution) and the mixture was transferred to a separatory funnel and after phase separation the organic phase was washed with water (100 mL, 5 vol).

Organic phase was distilled until 2 vol and heptane (80 mL, 4 vol) was added dropwise at room temperature. The suspension was stirred at the same temperature for 30 minutes and then filtered. The filter cake was washed with fresh heptane (1 vol). The solid was dried at 40°C under vacuum for 12 hours affording the title compound (5.9 g).
UPLC-MS m/z=246 [M+H]⁺.
¹H NMR (500 MHz, DMSO) δ 12.22 (s, 1H), 10.45 (s, 1H), 7.19 (d, J = 8.7 Hz, 1H), 6.82 (d, J = 2.6 Hz, 1H), 6.62 (dd, J = 8.7, 2.6 Hz, 1H), 3.73 (s, 3H), 3.50 - 3.39 (m, 1H), 2.79 - 2.55 (m, 4H), 2.41 - 2.28 (m, 1H), 2.14 - 1.96 (m, 1H).
Quiral HPLC: 99.5% ee.

### Example 6. One-pot preparation of (R)-2-(7-methoxy-1,2,3,4-tetrahydrocyclopenta[b]indol-3-yl)acetic acid (ETR05) from ethyl 2-(7-methoxy-1,4-dihydrocyclopenta[b]indol-3(2H)-ylidene)acetate (ETR03).

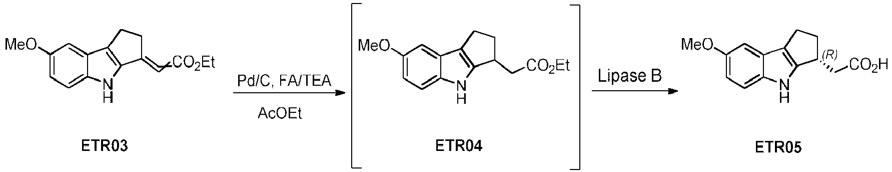

To a 500 mL round-bottom flask, ethyl 2-(7-methoxy-1,4-dihydrocyclopenta[b]indol-3(2H)-ylidene)acetate (38.64 g, 142.5 mmol) and AcOEt (773 mL, 20 vol) were added and the resulting mixture was stirred under nitrogen for 5 minutes. Then, 10% Pd/C (50% wet, 0.2 g/g, 7.73 g) and NEt₃ (59.6 mL, 427.5 mmol, 3 eq.) were added maintaining the temperature below 30°C. To the resulting mixture, formic acid (16.1 mL, 427.5 mmol, 3 eq.) was added dropwise maintaining the temperature below 30°C and the reaction was stirred at that temperature for 1h. The mixture was filtered through celite, washed with AcOEt (50 mL) and the organic phase was washed with water (3×100mL). The organic solvent was removed under pressure at 40°C affording ethyl 2-(7-methoxy-1,2,3,4-tetrahydrocyclopenta[b]indol-3-yl)acetate that was used in the next step without further purification.

**Preparation of 1.0 M buffer solution:** A solution of 1.16 g of potassium phosphate monobasic in purified water (8.52 mL) was added to a solution of 14.7 g of potassium phosphate dibasic in purified water (84 mL) and was stirred at room temperature for 5 minutes.

To a 1L round-bottom flask with ethyl 2-(7-methoxy-1,2,3,4-tetrahydrocyclopenta[b]indol-3-yl)acetate previously obtained, acetonitrile (386 mL, 10 vol), a portion of 1.0 M buffer previously prepared (30.9 mL, 0.8 vol) were added and the mixture was stirred at room temperature for 5 minutes. The resulting mixture was stirred (mechanic agitation, 160 rpm) under nitrogen and Lipase B, Candida Antarctica, immobilized (3.1 g, 0.08g/g) was added and the reaction was stirred at 40 °C overnight. The mixture was cooled at room temperature and contents were filtered to remove immobilized lipase. The round-bottom flask and solids were washed with acetonitrile (3x20 mL) and the combined filtrates were concentrated under pressure. To the crude, ethyl acetate (270 mL, 7 vol), NaHCOs (7% aqueous solution, 270 mL, 7 vol) and water (116 mL, 3 vol) were added and transferred to a separatory funnel. After phase separation was completed, the organic phase was extracted with a solution of NaHCOs (7% aqueous solution, 116 mL, 3 vol).

DCM (270 mL, 7 vol) was added to the reunited aqueous phases and the pH was adjusted between 4 and 5 by addition of HCl (18% aqueous solution) and the mixture was transferred to a separatory funnel and after phase separation the organic phase was washed with water (193 mL, 5 vol).

Organic solvent was removed at 40°C under vacuum affording the title compound (13.07 g).
UPLC-MS m/z=246 [M+H]⁺.
¹H NMR (500 MHz, DMSO) δ 12.22 (s, 1H), 10.45 (s, 1H), 7.19 (d, J = 8.7 Hz, 1H), 6.82 (d, J = 2.6 Hz, 1H), 6.62 (dd, J = 8.7, 2.6 Hz, 1H), 3.73 (s, 3H), 3.50 - 3.39 (m, 1H), 2.79 - 2.55 (m, 4H), 2.41 - 2.28 (m, 1H), 2.14 - 1.96 (m, 1H).
Quiral HPLC: 99.4% ee.

### Example 7. One-pot preparation of (R)-2-(7-methoxy-1,2,3,4-tetrahydrocyclopenta[b]indol-3-yl) acetic acid (ETR05) from ethyl 2-(7-methoxy-1,4-dihydrocyclopenta[b]indol-3(2H)-ylidene)acetate (ETR03) with epimerization of the unhydrolyzed (S) ester isomer for a second hydrolysis.

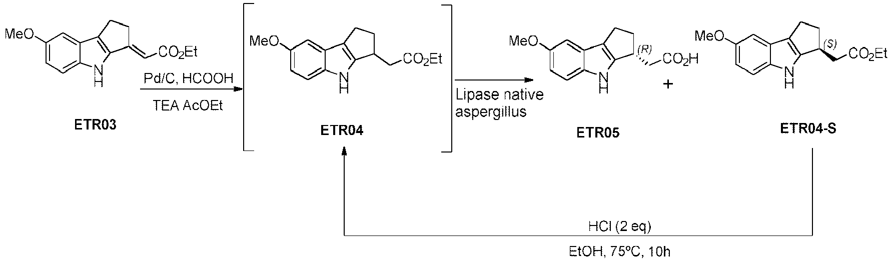

### Hydrogenation of ETR03

To a 1L round-bottom flask, ethyl 2-(7-methoxy-1,4-dihydrocyclopenta[b]indol-3(2H)-ylidene) acetate (30 g, 110 mmol), 10% Pd/C (50% wet, 6g) and EtOAc (600 mL, 20 vol) were added and the resulting mixture was stirred under nitrogen for 5 minutes. Then, NEt₃ (45.9 mL, 1.53 vol) was added and the system was inerted again with nitrogen. To the resulting mixture, formic acid (12.6 mL, 0.42 vol) was added dropwise maintaining the temperature below 30-32°C and the reaction was stirred at that temperature for 3h. The mixture was cooled at room temperature and the suspension was filtered through celite and washed with EtOAc (2×150 mL). The filtrate was acidified with aq HCl (3.5%, 240 ml, 8 vol) and the mixture was transferred to a separatory funnel. After phase separation was completed, the lower aqueous layer was drained. The organic phase was washed again with 2 × aq HCl (3.5%, 240 ml, 8 vol), 1 × NaHCOa aq solution (5%, 240 ml, 8 vol) and 1 × water (240 ml, 8 vol). The resulting organic phase was distilled under low pressure until a residue was formed, then acetonitrile (300 ml, 10 vol) was added to get a solution of ethyl 2-(7-methoxy-1,2,3,4-tetrahydrocyclopenta[b]indol-3-yl) acetate for use in the next step.

### Enzymatic Hydrolysis.- Part 1

Preparation of 1.0 M buffer solution: A solution of 1.16 g of potassium phosphate monobasic in purified water (8.52 mL) was added to a solution of 14.7 g of potassium phosphate dibasic in purified water (84 mL) and was stirred at room temperature for 5 minutes.

To the 1L round-bottom flask with the ACN solution of the previous step, a portion of 1.0 M buffer previously prepared (72 ml, 2.4 vol) and the immobilized native *Aspergillus sp.* lipase (NATE-1753) (1.8g, 0.06 g/g) were added, the resulting mixture was stirred (mechanic agitation, 170 rpm) under nitrogen at 40 °C overnight. The mixture was filtered to remove immobilized lipase, the reaction vessel and the lipase were washed with acetonitrile (60 ml, 2 vol), and the two-phase combined filtrates were decanted. Finally the organic phase was concentrated under reduced pressure.

To the crude residue, ethyl acetate (450mL, 15 vol) was added, and the solution was distilled partially until a final 10 vol. The organic phase was washed with 3 × NaHCO₃ 5% aq solution (12 vol each, 360 ml). The combined aqueous phases (with the carboxylic acid product) were kept in cold until next step.

### Epimerization of the remaining unhydrolyzed (S) ester isomer.

The EtOAc organic phase of the previous step (approx. 300 ml) was concentrated under reduced pressure, and to the crude residue formed, EtOH (300 ml, 10 vol) was added to get a solution.

To the ethanolic solution, aq 37% HCl (2.1 ml, 0.07 vol) was added and the reaction mixture was heated to 75°C for 10h, until the (S) epimerization was completed to a (R)/(S) mixture of ethyl 2-(7-methoxy-1,2,3,4-tetrahydrocyclopenta[b]indol-3-yl) acetate.

The reaction mixture was cooled and washed with an aq solution of NaHCO₃ 7% (300 ml, 10 vol) and water (300 ml, 10 vol). The EtOAc solvent of the organic phase was removed under reduced pressure and replaced by ACN (300 ml, 10 vol).

### Second Enzymatic Hydrolysis of the recovered (R)/(S) ester mixture. Part 2

The enzymatic hydrolysis was performed in the same way as it was explained in hydrolysis Part 1 to obtain the combined NaHCOs aqueous phase.

The two aqueous phases obtained in part 1 and part 2 are combined (2100 ml approx.), CH₂Cl₂ (600 ml, 20 vol) was added under stirring and the system was acidified with aq 37%HCl (from pH 8.9 until pH of 4.3, approx. 58 ml of HCl were added).

The two phases were decanted, the organic phase was partially concentrated to 10 vol and heptane (390 ml, 13 vol) was added. A precipitate was formed and the system was distilled again to 10 vol and refilled with another portion of heptane (390 ml, 13 vol).

The suspension was concentrated again to 10 vol and left stirring overnight, the suspension was filtered and the solid dried in a vacuum oven to get 15.87g of a solid (final yield of hydrogenation + enantiomeric hydrolysis: 59% yield).
Quiral HPLC: ≥99.0% ee.
Purity: 98%.
UPLC-MS m/z=246 [M+H]+.
¹H NMR (500 MHz, DMSO) δ 12.22 (s, 1H), 10.45 (s, 1H), 7.19 (d, J = 8.7 Hz, 1H), 6.82 (d, J = 2.6 Hz, 1H), 6.62 (dd, J = 8.7, 2.6 Hz, 1H), 3.73 (s, 3H), 3.50 - 3.39 (m, 1H), 2.79 - 2.55 (m, 4H), 2.41 - 2.28 (m, 1H), 2.14 - 1.96 (m, 1H).

### Example 8. Preparation of Methyl (R)-2-(7-hydroxy-1,2,3,4-tetrahydrocyclopenta[b]indol-3-yl)acetate (ETR07-Me) from (R)-2-(7-methoxy-1,2,3,4-tetrahydrocyclopenta[b]indol-3-yl) acetic acid (ETR05).

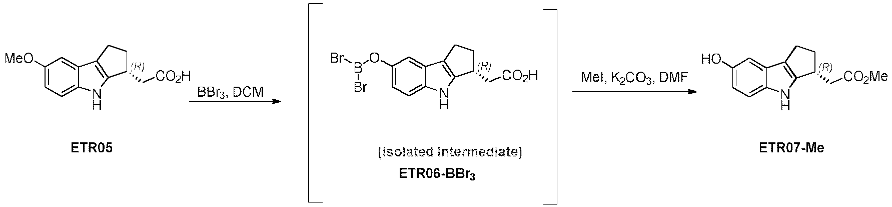

A solution of (R)-2-(7-methoxy-1,2,3,4-tetrahydrocyclopenta[b]indol-3-yl)acetic acid (30g, 122.4 mmol, ee:98.9%) in dichloromethane (900 mL, 30 vol) under N₂, was cooled at 0/-5°C and boron tribromide (36.0 mL, 381.5 mmol) was added dropwise and the temperature adjusted to 0/10°C. After 2 h, a solution of DCM (150 mL) and tBuOH (90 mL) was added adjusting the temperature below 20°C, and the mixture was stirred for about 1h. The suspension was filtered and washed with DCM (150 mL), and the filtrate was dissolved in DMF (600 mL).

K₂CO₃ (30 g) was added to the solution and was stirred at room temperature for about 15 minutes, then the mixture was heated to 45°C. Mel (6.3 mL) was added to the mixture and was stirred at 45°C for about 5 hours. The mixture was cooled down to room temperature, the solid was filtered and washed with IPE (600 mL). IPE (600 mL) and 2.4 L aqueous NaCl 10% were added to the aqueous phase. The organic phase was separated. The aqueous phase was extracted with IPE (600 mL).

The organic phase was washed with aqueous NaCl 10% (600 mL, twice) and was concentrated under vacuum. The solid obtained was stirred at room temperature for about 30 minutes. Heptane (200 ml) was added and the mixture was stirred at room temperature for about 60 minutes. The solid was filtered and washed with heptane (150 mL) and the solid was dried in a vacuum oven to get 19.2g (yield 64.0%) of a solid. No racemization was observed from the starting material.
Purity (wet cake): 96.8%.
UPLC-MS m/z=260 [M+H]⁺.
**ETR08-BBr₃:** ¹H NMR (500 MHz, DMSO) δ 10.28 (s, 1H), 7.07 (d, J = 8.6 Hz, H), 6.62 (d, J = 2.3 Hz, H), 6.49 (dd, J = 8.6, 2.4 Hz, H), 3.43 (m, 1H), 2.77 - 2.62 (m, 3H), 2.62 - 2.55 (m, 1H), 2.33 (dd, J = 16.0, 9.2 Hz, 1H), 2.05 (m, 1H).

### Example 9 (Comparative). Preparation of (R)-2-(7-hydroxy-1,2,3,4-tetrahydrocyclopenta[b]indol-3-yl)acetic acid (ETR06) with BBr₃ and quenching with NaOH aq.

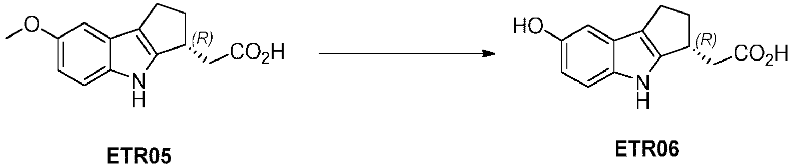

To a 100 mL round-bottom flask containing dichloromethane (15 mL, 5 vol) cooled at 0/-5°C was added boron tribromide (3.54 mL, 36.72 mmol) dropwise and the temperature adjusted to 0/10°C. This mixture was added to a solution of (R)-2-(7-methoxy-1,2,3,4-tetrahydrocyclopenta[b]indol-3-yl)acetic acid (3 g, 12.24 mmol) in dichlorometane (60 mL, 20 vol) and the content of the round-bottom flask was stirred at -5/0°C for 1 h.

The reaction mixture was added dropwise over a solution of NaOHaq (50%, 2.4 vol) in water (90 mL, 30 vol) maintaining the temperature below 10°C. The mixture was stirred for 10 mint and transferred to a separatory funnel. After phase separation, organic phase was extracted with NaOHaq (5%, 30 mL, 10 vol). The pH of reunited aqueous phases was adjusted between 4 and 5 by addition of HCl (2M aqueous solution) and the mixture was transferred to a separatory funnel and after phase separation the aqueous phase was extracted with AcOEt (30 mL, 10 vol).

Reunited organic phases were removed at 40°C under vacuum affording the title compound (4.44 g). Slight racemization was observed.
UPLC-MS m/z=232 [M+H]⁺.
¹H NMR (500 MHz, DMSO): δ 12.21 (s, 1H), 10.27 (s, 1H), 8.53 (s, 1H), 7.11 (d, J = 8.6 Hz, 1H), 6.67 (d, J = 2.4 Hz, 1H), 6.53 (dd, J = 8.6, 2.4 Hz, 1H), 3.56 - 3.41 (m, 1H), 2.81 - 2.56 (m, 4H), 2.37 (dd, J = 16.0, 9.0 Hz, 1H), 2.17 - 2.03 (m, 1H).
Quiral HPLC: 97.0% ee.

### Example 10 (Comparative). Preparation of (R)-2-(7-hydroxy-1,2,3,4-tetrahydrocyclopenta[b]indol-3-yl)acetic acid (ETR06) with BBr₃ and quenching with water.

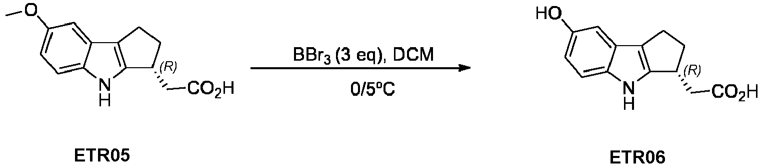

97.80% ee 91.30% ee To a 250 mL round-bottom flask containing dichloromethane (24 mL, 4 vol) cooled at 0/-5°C was added boron tribromide (7.1 mL, 73.56 mmol) dropwise and the temperature adjusted to 0/10°C. This mixture was added to a solution of (R)-2-(7-methoxy-1,2,3,4-tetrahydrocyclopenta[b]indol-3-yl)acetic acid (3 g, 12.24 mmol) in dichlorometane (60 mL, 20 vol) and the content of the round-bottom flask was stirred at -5/0°C for 1 h.

To the reaction mixture was added ice/water (10 mL) dropwise (exothermic reaction, reached temperature: 23 °C) and DCM (20 mL). The pH was adjusted to 8-9 with a solution of NaOHaq (50%) exothermic reaction (temperature below 23 °C). Water (20 mL) and DCM (20 mL) were added and transferred to a separatory funnel. The pH of aqueous phase was adjusted between 4 and 5 by addition of HCl (2M aqueous solution, 22 mL approx.) and the mixture was transferred to a separatory funnel and after phase separation, the organic phase was removed at 40°C under vacuum affording the desired compound (6.12 g). Substantial racemization was observed
UPLC-MS m/z=232 [M+H]+.
Quiral HPLC: 90.64% ee (95.32% R - 4.68% S).

### Example 11 (Comparative). Preparation of methyl (R)-2-(7-hydroxy-1,2,3,4-tetrahydrocyclopenta[b]indol-3-yl)acetate (ETR07-Me) in acid media.

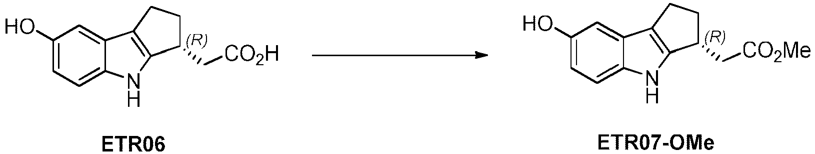

To a 100 mL round-bottom flask, (R)-2-(7-hydroxy-1,2,3,4-tetrahydrocyclopenta[b]indol-3-yl)acetic acid (4.6 g, 19.91 mmol), MeOH (36.8 mL, 8 vol), PTSA (0.56 g, 2.99 mmol) was added and the mixture was stirred at RT for 20 hours. The pH was adjusted between 8 and 9 by addition of NaHCOs (7% aqueous solution). MeOH was concentrated under pressure at 45°C and the resulting mixture was diluted with AcOEt (10 vol) and water (10 vol). The solution was transferred to a separatory funnel and after phase separation was completed, the organic phase was concentrated under vacuum. The crude was purified by chromatography column affording the title compound as a white solid (2.6 g). Slight racemization was observed. UPLC-MS m/z=245 [M+H]⁺.
¹H NMR (500 MHz, CDCl₃) δ 8.41 (s, 1H), 7.16 (d, J = 8.6 Hz, 1H), 6.89 (d, J = 2.5 Hz, 1H), 6.71 (dd, J = 8.7, 2.5 Hz, 1H), 5.09 (s, 1H), 3.79 (s, 3H), 3.64 - 3.49 (m, 1H), 2.91 - 2.69 (m, 4H), 2.56 (dd, J = 16.8, 10.8 Hz, 1H), 2.12 (dtd, J = 12.5, 4.2, 2.3 Hz, 1H). Quiral HPLC: 96.3% ee.

### Example 12. Preparation of ethyl (R)-2-(7-((4-cyclopentyl-3-(trifluoromethyl)benzyl)oxy)-1,2,3,4-tetrahydrocyclopenta[b]indol-3-yl)acetate (ETR08-Et).

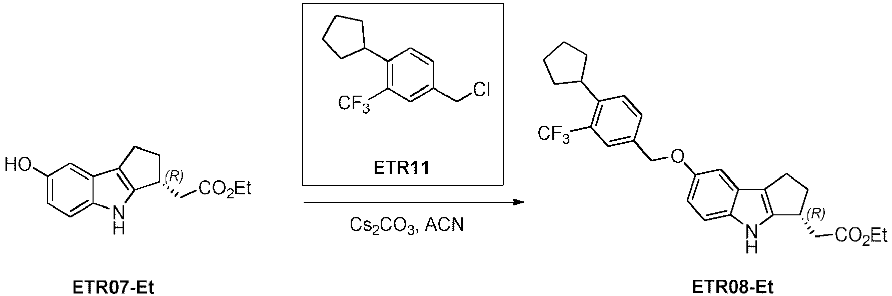

To a 50 mL round-bottom flask containing ethyl (R)-2-(7-hydroxy-1,2,3,4-tetrahydrocyclopenta[b]indol-3-yl)acetate (370 mg, 1.43 mmol) dissolved in acetonitrile (2.07 mL, 5.6 vol), cesium carbonate (600 mg, 1.86 mmol) was added and the mixture was heated to 40-45°C. To the previous mixture a solution of 4-(chloromethyl)-1-cyclopentyl-2-(trifluoromethyl)benzene (410 mg, 1.57 mmol) in acetonitrile (1.63 mL, 4.4 vol) was added and was heated at 60-65 °C overnight. The reaction mixture was cooled to 50-55 °C and filtered under nitrogen through a filter plate. The filter cake was washed with fresh hot (50-55 °C) acetonitrile (1.85 mL, 5 vol). The combined filtrates were concentrated by vacuum distillation affording the title compound (750 mg).
UPLC-MS m/z=486 [M+H]⁺.

### Example 13. Preparation of methyl (R)-2-(7-((4-cyclopentyl-3-(trifluoromethyl)benzyl)oxy)-1,2,3,4-tetrahydrocyclopenta[b]indol-3-yl)acetate (ETR08-Me).

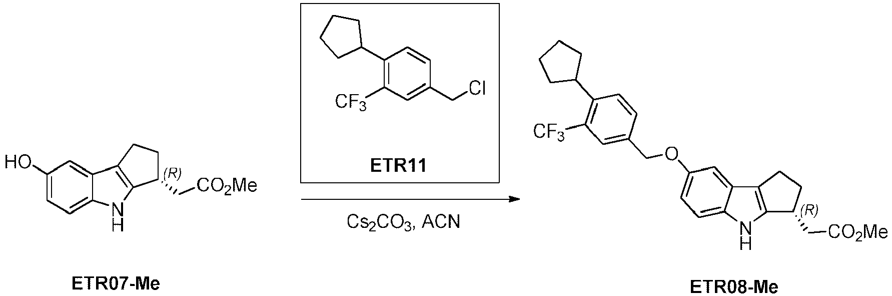

To a 50 mL round-bottom flask containing methyl (R)-2-(7-hydroxy-1,2,3,4-tetrahydrocyclopenta[b]indol-3-yl)acetate (700 mg, 3.22 mmol) dissolved in acetonitrile (4 mL, 5.6 vol), cesium carbonate (1.37 g, 4.19 mmol) was added and the mixture was heated to 40-45°C. To the previous mixture a solution of 4-(chloromethyl)-1-cyclopentyl-2-(trifluoromethyl)benzene (670 mg, 2.58 mmol) in acetonitrile (3 mL, 4.4 vol) was added and was heated at 60-65 °C overnight. The reaction mixture was cooled to 50-55 °C and filtered under nitrogen through a filter plate. The filter cake was washed with fresh hot (50-55 °C) acetonitrile (1.85 mL, 5 vol). The combined filtrates were concentrated and the crude was diluted with AcOEt (10 vol) and water (10 vol). After phase separation, organics were removed by vacuum distillation affording the title compound (1.4 g).
UPLC-MS m/z=471 [M+H]⁺.

### Example 14. Preparation of Methyl (R)-2-(7-((4-cyclopentyl-3-(trifluoromethyl)benzyl)oxy)-1,2,3,4-tetrahydrocyclopenta[b]indol-3-yl)acetate (ETR08-Me).

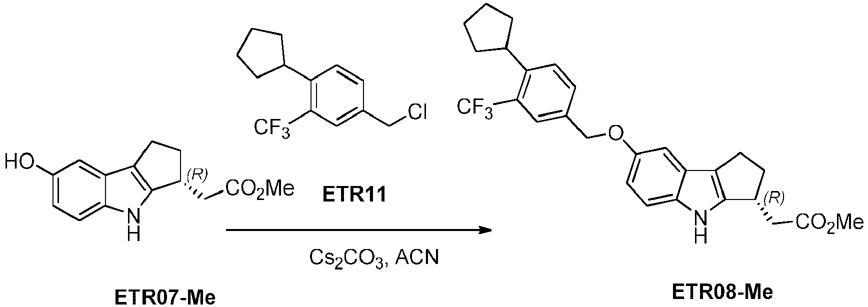

To a 50 mL round-bottom flask containing (R)-2-(7-hydroxy-1,2,3,4-tetrahydrocyclopenta[b]indol-3-yl) acetate (25 g, 102 mmol) dissolved in acetonitrile (150 mL, 6 vol), Cs₂CO₃ (36.5 g, 112 mmol, 1.1 eq) was added and the mixture was inertized under N₂ and heated to 50°C. To the reaction mixture a solution of 4-(chloromethyl)-1-cyclopentyl-2-(trifluoromethyl) benzene (28 g, 107 mmol, 1.05 eq) in acetonitrile (100 mL, 4 vol) was added in 5 min, the resulting mixture was heated at 50 °C overnight and after that period it was cooled to room temperature and filtered. The filter cake was washed with acetonitrile (100 mL, 4 vol) and the combined filtrates were concentrated by vacuum distillation and the residue obtained was dissolved again with MeOH (200 ml, 8 vol) and the solution was concentrated again under vacuum. Finally, more MeOH was added (200 ml, 8 vol) and distilled partially until a final Volume of 4 vol.

At room temperature, the system was seeded with a sample of ETR08-Me and kept stirring until a thick suspension was formed, then, two additional volumes of MeOH ere added (50 ml) and kept shaking for three hours more, the system was cooled to 0/5°C for at least 30 minutes and filtered.

The filter cake was washed with cold MeOH (50 ml, 2 vol) and the solid obtained was dried to get 27 g of ETR 08 (>99.8% ee, 99% purity) with a Molar yield of 56%.

An additional amount of product can be recovered by using the mother liqueurs in another crystallization in the next reaction process.
UPLC-MS m/z=486 [M+H]⁺.
¹H NMR (500 MHz, Acetone) δ 9.76 (s, 1H), 7.77 (d, J = 1.8 Hz, 1H), 7.73 (dd, J = 8.1, 1.8 Hz, 1H), 7.63 (d, J = 8.2 Hz, 1H), 7.27 (d, J = 8.7 Hz, 1H), 7.01 (d, J = 2.4 Hz, 1H), 6.78 (dd, J = 8.7, 2.5 Hz, 1H), 5.16 (s, 2H), 3.69 (s, 3H), 3.61 -3.52 (m, 1H), 3.37 (m, 1H), 2.81 - 2.64 (m, 4H), 2.57 (dd, J = 16.2, 7.4 Hz, 1H), 2.16 - 2.08 (m, 2H), 1.94 - 1.84 (m, 2H), 1.77 - 1.61 (m, 4H).

### Example 15. Preparation of (R)-2-(7-((4-cyclopentyl-3-(trifluoromethyl)benzyl)oxy)-1,2,3,4-tetrahydrocyclopenta[b]indol-3-yl)acetic acid (ETR09).

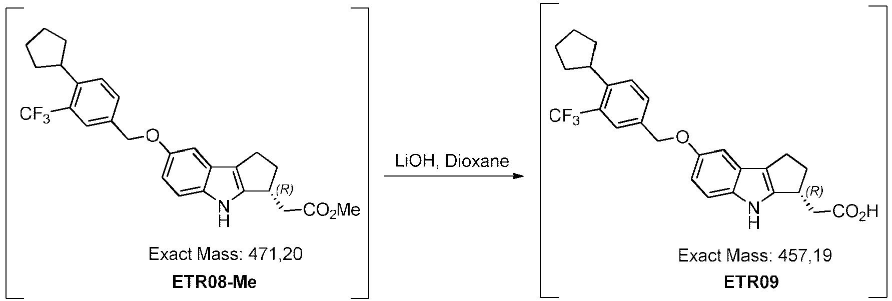

To a 100 mL round-bottom flask containing (R)-2-(7-((4-cyclopentyl-3-(trifluoromethyl)benzyl)oxy)-1,2,3,4-tetrahydrocyclopenta[b]indol-3-yl)acetate (700 mg, 1.43 mmol), 1,4-dioxane (5.6 mL, 8 vol) and LiOH (2N, 2.14 mL, 3.1 vol) were added and the mixture was stirred at 50°C under nitrogen for 3h. The reaction mixture was cooled at room temperature and the organic was removed under vacuum. The mixture was diluted with AcOEt (7 mL, 10 vol) and water (7 mL, 10 vol) and the pH was adjusted between 3 and 5 by addition of commercial HCl (37%). The solution was transferred to a separatory funnel and after phase separation was completed, the organic phase was concentrated under vacuum affording the title compound.
UPLC-MS m/z=458 [M+H]⁺.

### Example 16. Preparation of (R)-2-(7-((4-cyclopentyl-3-(trifluoromethyl)benzyl)oxy)-1,2,3,4-tetrahydrocyclopenta [b]indol-3-yl)acetic acid (ETR09) and final Etrasimod (Arginine salt) (ETR10).

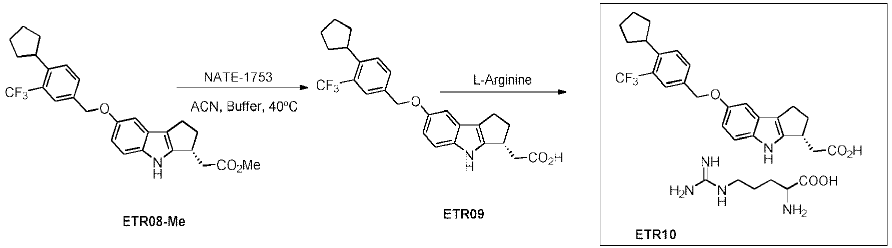

**Preparation of 1.0 M buffer solution:** A solution of 2.32 g of potassium phosphate monobasic in purified water (17 mL) was added to a solution of 28.4 g of potassium phosphate dibasic in purified water (168 mL) and was stirred at room temperature for 5 minutes.

### Enzymatic Hydrolysis.-

To a solution of ETR08-Me (21g, 44.56 mmol) in ACN (672 ml, 32 vol), a 1.0 M Buffer solution previously prepared (168 ml, 8V) was added and the immobilized NATE-1753 Lipase (1.26 g, 0.06 g/g of Native Aspergillus)) was added. The resulting mixture was stirred (mechanic agitation, 170 rpm) under nitrogen at 40 °C for 20h. The mixture was cooled to Room temperature and the Lipase was removed by filtration and washed with acetonitrile (42 ml, 2 vol).

Over the reaction mixture, a 5M solution of citric acid was added until a pH of about 4.8 (10 ml) and the two-phase combined filtrates were decanted. Finally, the organic phase was concentrated under reduced pressure.

To the crude residue, ethyl acetate (168mL, 8 vol) was added and washed with water (105 ml, 5 vol) and brine (105 ml, 5 vol). The organic phase was distilled under reduced pressure until residue and ACN was added and distilled again, giving rise to an Oil (21.6g, 106% Molar yield).

### Etrasimod Arginine salt.

To a solution of ETR09 from the previous step in ACN (195 ml, 9.3 vol) kept at 55°C, a hot solution (60°C) of L-Arginine (7.45g, 42.8 mmol, 0.96 eq) in water (18,8 ml, 0.9V) was slowly added (1ml/1.5 min) in 45 min.

The system was kept 30 min at 55°C with stirring and then, it is slowly cooled down to 25°C (30 min). The suspension formed was filtered and washed with ACN (2 x 5 vol, 2 x 105 ml). The filter cake was washed again with AcOEt (2 x 63 ml, 2 x 3 vol), and the solid obtained was dried giving rise to 25.3g (molar yield: 90% from ETR08-Me).
Purity: ≥99%.
Chiral HPLC: ≥99.8% ee.

### Example 17. Preparation of crystalline L-Arginine salt of (R)-2-(7-(4-cyclopentyl-3-(trifluoromethyl)benzyloxy)-1,2,3,4-tetrahydrocyclopenta[b]indol-3-yl)acetate (ETR10).

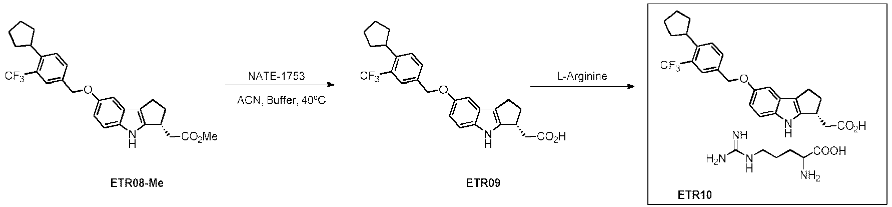

A phosphate buffer solution was prepared as follows:
27.6 of potassium phosphate dibasic were dissolved in 157.5 mL of water.
2.18 g of potassium phosphate monobasic were dissolved in 15.9 mL of water.

The two solutions were mixed together, affording the potassium phosphate buffer solution (pH = 7.7±3).

(R)-Methyl 2-(7-(4-cyclopentyl-3-(trifluoromethyl)benzyloxy)-1,2,3,4-tetrahydrocyclopenta[b]indol-3-yl)acetate (21g , 44.56 mmol) was dissolved in 672 mL of acetonitrile. Then 168 mL of a previously prepared potassium phosphate buffer solution was added, followed by 1.25g of Native Aspergillus sp. Lipase (immobilized) (NATE-1753). The resulting mixture was stirred under nitrogen at the temperature of 40±5 °C, during 20h. The mixture was then cooled at 25±5 °C and the pH adjusted between 4 and 5 by addition of an aqueous solution of citric acid 5M. The resulting suspension was filtered to remove the immobilized lipase and the phosphate and citrate salts. The filter cake was washed with 2 portions of 100 mL of acetonitrile. Separation of phases was observed in the resulting mother liquors. The lower aqueous phase was discharged and the organic phase was concentrated by vacuum distillation at a temperature of 40±5 °C. The residue was dissolved by addition of 168 mL of ethyl acetate, and washed with 105 mL of water. The organic phase was separated and washed with 105 mL of a solution of sodium chloride 20 % w/w. After separation, the organic phase was concentrated by vacuum distillation at a temperature of 40±5 °C. The residue was dissolved by addition of 195 mL of acetonitrile and the amount of (R)-2-(7-(4-cyclopentyl-3-(trifluoromethyl)benzyloxy)-1,2,3,4-tetrahydrocyclopenta[b]indol-3-yl)acetic acid was quantified by UPLC.

An aqueous solution of L-Arginine (prepared by dissolving 7.45 g of L-Arginine in 18.8 mL of water at 55±5 °C) was added dropwise, during a period of 45±5 min, over the solution of (R)-2-(7-(4-cyclopentyl-3-(trifluoromethyl)benzyloxy)-1,2,3,4-tetrahydrocyclopenta[b]indol-3-yl)acetic acid at the temperature of 55±5 °C. After the addition was completed, the resulting suspension was stirred at 55±5 °C during 60 min. Then cooled at 25±5 °C at an approximate rate of 0.5 °C/min, and stirred at 25±5 °C during 30 min. The solid was filtered and the wet cake in the filter was washed with 2 portions of 100 mL of acetonitrile. Then, it was dried under reduced pressure at the temperature of 40±5 °C, obtaining 25.3 g (129% w/w) of L-Arginine salt of (R)-2-(7-(4-cyclopentyl-3-(trifluoromethyl)benzyloxy)-1,2,3,4-tetrahydrocyclopenta[b]indol-3-yl)acetic acid, as white solid.

HPLC purity ≥99.0%, Enantiomeric purity ≥99.8%

**Figures 4**, **5** **and** **6** show, respectively, the SEM imagen, the PXRD pattern and DSC analysis of the crystalline etrasimod arginine obtained.
**Specific Surface Area (BET method):** 9.80 m²/g, PSD D90 76.1 µm
**PSD Equipment:** Malvern Mastersizer 3000S. HydroMV measurement
**Specific Surface Area (BET method) equipment:** MicromeriticsTristar II 3020.

### Example 18. Preparation of Etrasimod-L-arginine amorphous form (ETR10).

2.0 g of Etrasimod-L-arginine were loaded into a flask, and then 24 mL of purified water were added.

The suspension was stirred at a temperature of 20-25 °C, and then 6 mL of acetonitrile were added.

The mixture was stirred at a temperature of 20-25 °C until completely dissolved.

The solution was filtered through a 0.45 µm filter, to remove insoluble particles.

14 ml aliquots of the sample solution of Etrasimod-L-arginine in water/acetonitrile were separated and packaged in glass bottles. The glass bottles were transferred to the freeze dryer (LYOBETA, 4PS, TELSTAR) and the lyophilized cycle was started

| **Temperature** | **Vacuum** | **Time** |
|---|---|---|
| -60°C | | 8h |
| -30°C | 500 µbar | 1.5h |
| -30°C | 0 µbar | 20h |
| 17°C | | 13h |

**Figure 7** shows the PXRD pattern of the amorphous etrasimod arginine obtained.

### Example 19. Comparison of the preparation of etrasimod L-arginine according to the prior art process (WO2011094008 A1) and according to one embodiment of the present invention.

| Route | Starting Material | Overall molar yield | Overall weight yield |
|---|---|---|---|
| Fig. 2 (Present invention) | 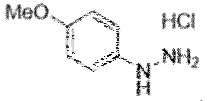 | 14% | 50% |
| Fig. 1 (Prior art) | 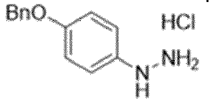 | 10% | 25% |

## Claims

1. A process for preparing preparing a compound of Formula (VIII) or a salt or solvate thereof 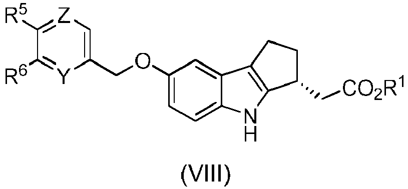 wherein
R¹ is an organic group forming an ester with the carboxyl group to which it is attached;
Y is N or CR³;
Z is N or CR⁴; and
R³, R⁴, R⁵ and R⁶ are each independently selected from the group consisting of H, C₁-C₆ alkyl, C₂-C₆ alkenyl, C₃-C₇ cycloalkyl, C₆-C₁₀ aryl, (C₆-C₁₀)aryl(C₁-C₆)alkyl, 3- to 10-membered heterocyclyl, 3- to 10-membered heteroaryl, C₁-C₆ alkoxy, C₁-C₆ alkylamino, C₁-C₆ alkylsulfonyl, C₁-C₆ alkylthio, carboxamide, cyano, C₃-C₇ cycloalkoxy, C₁-C₆ haloalkoxy, C₁-C₆ haloalkyl, and halogen, wherein said C₁-C₆ alkyl and C₁-C₆ alkoxy are each optionally substituted with one or two substituents selected from C₃-C₇ cycloalkyl and halogen;
from a compound of Formula (IV) or a salt or solvate thereof 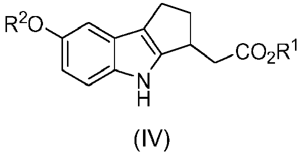
wherein
R¹ is as defined for the compound of Formula (VIII); and
R² is selected from the group consisting of C₁-C₆ alkyl and C₃-C₇ cycloalkyl, wherein said C₁-C₆ alkyl and C₃-C₇ cycloalkyl are each optionally substituted with one or more substituents selected from the group consisting of C₁₋₆ alkyl, C₁₋₆ alkoxy, halogen, C₁₋₆ haloalkyl, C₁₋₆ haloalkoxy, nitro and cyano;
said process comprising:
(a1) enantioselective enzymatic hydrolysis of the compound of Formula (IV) or a salt or solvate thereof in the presence of a lipase selected *Thermomyces lanuginosus* lipase and *Candida antarctica* lipase B to provide a compound of Formula (V) or a salt or solvate thereof 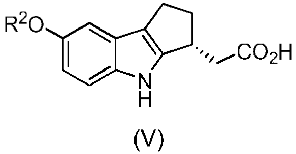 wherein
R² is as defined for the compound of Formula (IV);
(b1) hydroxyl deprotection of the compound of Formula (V) or a salt or solvate thereof with boron tribromide, followed by quenching with a tertiary alcohol, to provide a compound of Formula (VI-BBr₃) and/or (VI) or a salt or solvate thereof 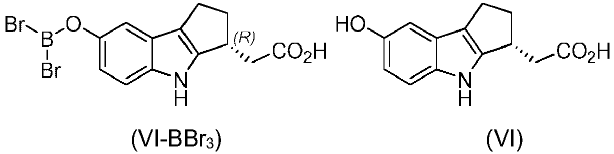 esterification of the compound of Formula (VI-BBr₃) and/or (VI) or a salt or solvate thereof in basic media to provide a compound of Formula (VII) or a salt or solvate thereof 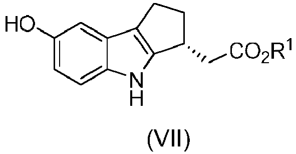 wherein
R¹ is as defined for the compound of Formula (VIII); and
(c) reaction of the compound of Formula (VII) or a salt or solvate thereof with a compound of Formula (XI) or a salt or solvate thereof 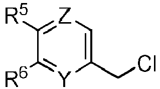 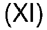 wherein
Y, Z, R⁵ and R⁶ are as defined for the compound of Formula (VIII);
to provide the compound of Formula (VIII) or a salt or solvate thereof;
or said process comprising:
(a2) hydroxyl deprotection of the compound of Formula (IV) or a salt or solvate thereof in the presence of a hydroxyl deprotection agent to provide a compound of Formula (XII) or a salt or solvate thereof; 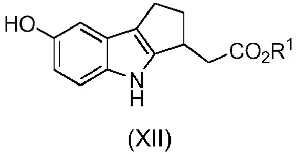 wherein
R¹ is as defined for the compound of Formula (VIII);
(b2) enantioselective enzymatic hydrolysis of a compound of Formula (XII) or a salt or solvate thereof in the presence of a lipase selected from *Thermomyces lanuginosus* lipase and *Candida antarctica* lipase B to provide a compound of Formula (VII) or a salt or solvate thereof 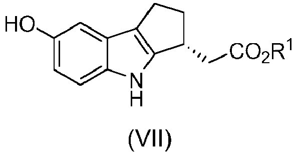 wherein
R¹ is as defined for the compound of Formula (VIII);
(c) reaction of the compound of Formula (VII) or a salt or solvate thereof with a compound of Formula (XI) or a salt or solvate thereof 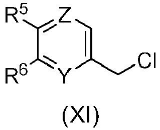 wherein
Y, Z, R⁵ and R⁶ are as defined for the compound of Formula (VIII);
to provide the compound of Formula (VIII) or a salt or solvate thereof.

2. The process according to claim 1, wherein the compound of Formula (IV) or a salt or solvate thereof is obtained by reduction of a compound of Formula (III) or a salt or solvate thereof 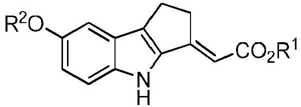 wherein
R¹ and R² are as defined for the compound of Formula (IV).

3. The process according to claim 2, wherein the reduction is conducted in the presence of a reduction agent, a catalyst and a solvent, wherein
- the reduction agent preferably comprises formic acid and a base, more preferably formic acid and trimethylamine;
- the catalyst preferably comprises palladium, more preferably is about 10% palladium on carbon; and
- the solvent is preferably selected from the group consisting of ethyl acetate, tetrahydrofuran and acetone, more preferably tetrahydrofuran or ethyl acetate.

4. The process according to claim 2 or 3, wherein the compound of Formula (III) or a salt or solvate thereof is obtained by reaction of a compound of Formula (I) or a salt or solvate thereof 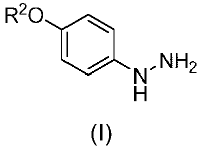 wherein
R² is as defined for the compound of Formula (IV);
with a compound of Formula (II) or a salt or solvate thereof 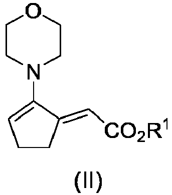
wherein
R¹ is as defined for the compound of Formula (VIII).

5. The process according to any one of claims 1 to 4, wherein the enantioselective enzymatic hydrolysis is conducted in the presence of a lipase selected from *Thermomyces lanuginosus* lipase and *Candida Antarctica* lipase B, a solvent and a buffer, wherein
- the lipase is preferably immobilized native *Aspergillus sp.* Lipase, immobilized *Candida antarctica* lipase B recombinant from yeast or immobilized *Candida antarctica* lipase B recombinant *Aspergillus oryzae;*
- the solvent is preferably selected from the group consisting of dimethylformamide (DMF), dimethylacetamide (DMA), dimethylsulfoxide (DMSO), tetrahydrofuran (THF), or acetonitrile, more preferably acetonitrile; and
- the buffer is preferably a phosphate buffer, more preferably a phosphate buffer at a pH of about 7.5.

6. The process according to any one of claims 1 to 5, wherein the undesired (S)-isomer ester of Formula (IV) or (XII) that remains non-hydrolyzed after the enantioselective enzymatic hydrolysis is epimerized and subjected to a further enantioselective hydrolysis.

7. The process according to any one of claims 1 to 6, wherein the hydroxyl deprotection agent is boron tribromide.

8. The process according to any one of claims 1 to 7, wherein the tertiary alcohol used for quenching is selected for instance from the group consisting of t-butyl alcohol, t-amyl alcohol, t-hexyl alcohols, t-heptyl alcohols and the t-octyl alcohols; more preferably, t-butyl alcohol.

9. The process according to any one of claims 1 to 8, wherein the esterification is conducted in the presence of a C₁-C₆ alkyl halide or a (C₆-C₁₀)aryl(C₁-C₆)alkyl halide and a carbonate base.

10. The process according to any one of claims 1 to 9, wherein the reaction of the compound of Formula (VII) or a salt or solvate thereof with a compound of Formula (XI) or a salt or solvate thereof is conducted in the presence of a base and a solvent, wherein
- the base is preferably selected from a carbonate base, more preferably cesium carbonate; and
- the solvent is preferably selected from the group consisting of acetone and acetonitrile.

11. The process according to any one of claims 1 to 10, wherein
R¹ is selected from the group consisting of C₁-C₆ alkyl, C₃-C₇ cycloalkyl, C₆-C₁₀ aryl, and (C₆-C₁₀)aryl(C₁-C₆)alkyl, preferably R¹ is C₁-C₆ alkyl or (C₆-C₁₀)aryl(C₁-C₆)alkyl, more preferably methyl, ethyl or benzyl;
R² is C₁-C₆ alkyl, preferably R² is methyl or ethyl;
Y is N or CR³, wherein R³ is H or C₁-C₆ haloalkyl, preferably R³ is H;
Z is N or CR⁴, wherein R⁴ is selected from the group consisting of H, cyano, C₁-C₆ haloalkoxy and C₁-C₆ haloalkyl; preferably R⁴ is H;
R⁵ is selected from the group consisting of is selected from the group consisting of H, C₁-C₆ alkyl, C₃-C₇ cycloalkyl, 3- to 10-membered heterocyclyl, C₁-C₆ alkoxy, C₁-C₆ haloalkoxy, C₁-C₆ haloalkyl, and halogen; preferably, R⁵ is cyclopentyl; and R⁶ is selected from the group consisting of H, cyano, C₁-C₆ haloalkoxy and C₁-C₆ haloalkyl; preferably, R⁶ is trifluoromethyl.

12. The process according to any one of claims 1 to 11, which further comprises the hydrolysis of the compound of Formula (VIII) or a salt or solvate thereof into compound of Formula (IX) or a salt or solvate thereof 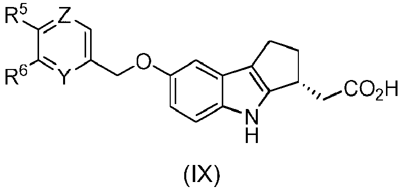 wherein
R¹, Y, Z, R³, R⁴, R⁵ and R⁶ are as defined for the compound of Formula (VIII).

13. The process according to claim 12, wherein the hydrolysis is a basic hydrolysis, an enzymatic hydrolysis or a hydrogenolysis;
wherein the basic hydrolysis is preferably conducted in the presence of a base and a solvent, wherein
- the base is preferably a hydroxide, more preferably lithium hydroxide; and
- the solvent is preferably selected from the group consisting of dioxane, ethanol and THF, more preferably is dioxane;
wherein the enzymatic hydrolysis is conducted in the presence of a lipase and a solvent, wherein
- the lipase is preferably *Thermomyces lanuginosus* lipase or *Candida antarctica* lipase B; and
- the solvent is preferably selected from the group consisting of dimethylformamide (DMF), dimethylacetamide (DMA), dimethylsulfoxide (DMSO), tetrahydrofuran (THF), or acetonitrile, more preferably is acetonitrile;
wherein the hydrogenolysis is conducted in the presence of a catalyst.

14. The process according to any one of claims 12 and 13, wherein the compound of Formula (IX) obtained is etrasimod, which is preferably converted into a salt thereof, preferably the L-arginine salt of etrasimod.

15. A compound which is selected from:
